# EUROPEAN PATENT APPLICATION

(11) **EP 4 755 411 A1**
(43) Date of publication of application: **10.06.2026**
(21) Application number: 24851557.9
(22) Date of filing: 18.07.2024
(51) Int. Cl.: A61M 1/36

(54) **DIAPHRAGM-TYPE PRESSURE MEASURING CHAMBER**

(30) Priority: 04.08.2023 JP 2023127797
(71) Applicant: Nipro Corporation, Settsu-shi, Osaka 566-8510 (JP)
(72) Inventor: YAMAGATA, Minami, Settsu-shi, Osaka 566-8510 (JP); YAMAGUCHI, Takeshi, Settsu-shi, Osaka 566-8510 (JP); ASAKA, Shinya, Settsu-shi, Osaka 566-8510 (JP); ESHIMA, Takashi, Settsu-shi, Osaka 566-8510 (JP)
(74) Representative: Dantz, Jan Henning
(86) International application number: PCT/JP2024/025742
(87) International publication number: WO 2025/033128

(57) **Abstract**

A pressure measuring chamber (1) includes: a first case (11) having a peripheral portion provided with a first connection portion (60); a second case (12) forming a chamber space (13) between the second case (12) and the first case (11) and having a peripheral portion provided with a second connection portion (70) connected to the first connection portion (60); and a flexible dome-shaped diaphragm (15) partitioning the chamber space (13) into a first chamber (13a) on a blood side and a second chamber (13b) on a pressure sensor side. The diaphragm (15) has a flexible membrane (40) deformable in a first direction according to a pressure difference between the first chamber (13a) and the second chamber (13b) and a support frame (50) provided on a peripheral portion of the flexible membrane (40) and fixed between the first connection portion (60) and the second connection portion (70). The support frame (50) has a higher rigidity than the flexible membrane (40).

## Description

### Technical Field

The present invention relates to diaphragm-type pressure measuring chambers for measuring a pressure of blood flowing through a blood circuit.

### Background Art

There are blood purification therapies in which blood of a patient is extracorporeally circulated and purified during the circulation. Dialysis therapy is known as an example of such therapies. In dialysis therapy, blood removed from a patient is introduced to a dialyzer which then substitutes unwanted components in the blood with a useful component in a dialysate and also filters the blood, and the processed blood is returned into the patient again. Also, in such dialysis therapy, blood pressures are measured at respective positions in a blood circuit, through which blood passes, in order to prevent damage to useful blood cells and blood agglutination.

The measurement of each of the pressures at the respective positions in the blood circuit is performed by, for example, measuring an air pressure in a drip chamber located halfway through the circuit. However, there are concerns that contact of blood with air in the drip chamber might make it easy for a thrombus to be formed, blood in the drip chamber might come into contact with a pressure sensor, or other troubles might occur. In view of the concerns, a chamber device in which a blood chamber for blood to flow therein and an air chamber leading to a pressure sensor are separated from each other by a flexible partition membrane has been proposed (see Patent Literature 1).

### Citation List

### Patent Literature

PTL 1: JP 2008-051663A

### Summary of Invention

### Technical Problem

Meanwhile, chambers in blood circuits are for measuring low pressures of -300 to 500 mmHg. Thus, the flexibility of the partition membrane is preferably high in order to increase the accuracy of pressure measurement. However, such a highly flexible partition membrane is easily deformed, and it is difficult to keep the shape thereof unchanged.
Therefore, such a partition membrane is difficult to handle. In particular, a chamber device including a dome-shaped partition membrane is assembled by sandwiching a peripheral edge portion of the partition membrane between upper and lower cases, and work for the assembly is difficult since the shape of the partition membrane is unstable. If a wrinkle or a strain is generated on the partition membrane during the assembly, the wrinkle or the strain might influence the accuracy of pressure measurement.

Considering this, an object of the present disclosure is to provide a diaphragm-type pressure measuring chamber that is favorable in terms of ease of assembly and can be appropriately assembled.

### Solution to Problem

A diaphragm-type pressure measuring chamber according to a first aspect of the present disclosure is a diaphragm-type pressure measuring chamber for measuring a pressure of blood in a blood circuit, the diaphragm-type pressure measuring chamber including: a first case having a peripheral portion provided with a first connection portion; a second case forming a chamber space between the second case and the first case and having a peripheral portion provided with a second connection portion connected to the first connection portion; and a flexible dome-shaped diaphragm partitioning the chamber space into a first chamber on a blood side and a second chamber on a pressure sensor side, wherein the first chamber is formed between the first case and the diaphragm and is in communication with a blood flow path in the blood circuit, the diaphragm has a flexible membrane deformable in a first direction according to a pressure difference between the first chamber and the second chamber and a support frame provided on a peripheral portion of the flexible membrane and fixed between the first connection portion and the second connection portion, and the support frame has a higher rigidity than the flexible membrane.

This can make it easy for a membrane portion of the diaphragm to be deformed, and meanwhile, can make it not easy for a peripheral edge of the diaphragm to be deformed, whereby influence of deformation of the membrane portion of the diaphragm on the peripheral edge of the diaphragm can be decreased. Consequently, the dome-shaped diaphragm having the highly rigid support frame is easily handled, and, even when the dome-shaped membrane portion is deformed in an assembly process, the shape of the peripheral edge of the diaphragm is stable. Thus, the pressure measuring chamber can be appropriately assembled with the diaphragm being sandwiched between the first case and the second case. Therefore, the dome-shaped diaphragm can be made less likely to sustain a wrinkle or a strain.

A pressure measuring chamber according to a second aspect of the present disclosure may be the pressure measuring chamber according to the first aspect, wherein at least one of the first connection portion and the second connection portion is provided with an insertion groove into which the support frame is inserted in the first direction.

Consequently, when the diaphragm is fixed between the first case and the second case, the support frame is inserted into the insertion groove of the first case or the second case, whereby the diaphragm is easily positioned with respect to the first case or the second case, and the stability of positional accuracy is improved.

A pressure measuring chamber according to a third aspect of the present disclosure may be the pressure measuring chamber according to the second aspect, wherein the first connection portion and the second connection portion form an accommodation space accommodating the support frame, and the flexible membrane has a seal portion sandwiched and compressed between the first connection portion and the second connection portion. The compression may be realized by sandwiching the seal portion between surfaces of the first connection portion and the second connection portion, but provision of a compressing protrusion on at least one of the first connection portion and the second connection portion enables efficient formation of the seal portion.

In this case, the rigidity of the support frame can be made higher and the stability of the peripheral edge of the diaphragm can be made higher, than in a case where the support frame serves as a seal portion. In a case where the support frame and the flexible membrane are formed through integral molding, a stress due to a positional shift of the flexible membrane is blocked by a portion compressed by the first connection portion and the second connection portion at the seal portion. Thus, the stress becomes less likely to be transmitted to a fixation portion between the support frame and the flexible membrane. Therefore, peeling from the support frame at the fixation portion can be inhibited.

A pressure measuring chamber according to a fourth aspect of the present disclosure may be the pressure measuring chamber according to the third aspect, wherein the first case is engaged with the second case, and the accommodation space is formed with a dimension thereof in the first direction being longer than a dimension in the first direction of the support frame to provide a gap between the support frame and the first connection portion or the second connection portion inside the accommodation space.

Formation of a gap between the support frame and the first connection portion or the second connection portion inside the accommodation space enables a decrease in a risk that, when the first case is fixed to the second case, imperfection in the fixation occurs owing to the support frame. The engagement encompasses physical fitting by means of a recess and a protrusion or the like and fixation by means of an adhesive or integral molding.

A pressure measuring chamber according to a fifth aspect of the present disclosure may be the pressure measuring chamber according to the second aspect, wherein the support frame has a protective portion located radially outward of the flexible membrane.

Consequently, when the peripheral edge portion of the diaphragm is inserted into the insertion groove, the flexible membrane can be inhibited from rubbing on a wall surface of the insertion groove, and the flexible membrane can be easily inserted into the insertion groove in an appropriate state.

A pressure measuring chamber according to a sixth aspect of the present disclosure may be the pressure measuring chamber according to the fifth aspect, wherein the flexible membrane has an embedded portion having an inner surface and an outer surface in a second direction and embedded in the support frame with the inner surface and the outer surface being fixed to the support frame.

Consequently, a fixation surface can be made large, whereby the support frame can more stably hold the flexible membrane. In addition, the area of the flexible membrane exposed at the peripheral edge of the diaphragm can be made small, whereby it can be made less likely for unintentional external force to be applied to the flexible membrane.

A pressure measuring chamber according to a seventh aspect of the present disclosure may be the pressure measuring chamber according to the sixth aspect, wherein the protective portion has an extended portion extending in the first direction beyond the outer surface of the embedded portion of the flexible membrane.

Consequently, an outer peripheral edge of the flexible membrane is inhibited from coming into contact with a bottom surface and a top surface of the first connection portion and the second connection portion, whereby it can be made less likely for unintentional stress to be applied to the outer peripheral edge of the flexible membrane during mounting.

A diaphragm-type pressure measuring chamber according to an eighth aspect of the present disclosure is a diaphragm-type pressure measuring chamber for measuring a pressure of blood in a blood circuit, the diaphragm-type pressure measuring chamber including: a first case having a peripheral portion provided with a first connection portion; a second case forming a chamber space between the second case and the first case and having a peripheral portion provided with a second connection portion connected to the first connection portion; and a flexible diaphragm partitioning the chamber space into a first chamber on a blood side and a second chamber on a pressure sensor side, wherein the first chamber is formed between the first case and the diaphragm and is in communication with a blood flow path in the blood circuit, the diaphragm has a flexible membrane having a surface area larger than a cross-sectional area of the chamber space and having a three-dimensional shape at least partially protruding in a first direction, the flexible membrane being deformable in the first direction according to a pressure difference between the first chamber and the second chamber, and a support frame provided on a peripheral portion of the flexible membrane and fixed between the first connection portion and the second connection portion, and the support frame has a higher rigidity than the flexible membrane.

Consequently, the diaphragm having the highly rigid support frame can be easily handled, and the pressure measuring chamber can be assembled with the diaphragm being sandwiched between the first case and the second case to be located at an appropriate position. Therefore, defects which are a wrinkle or a strain unique to such a three-dimensional diaphragm can be made less likely to occur.

### Advantageous Effects of Invention

The present disclosure makes it possible to provide a diaphragm-type pressure measuring chamber that is favorable in terms of ease of assembly and can be appropriately assembled.

### Brief Description of Drawings

FIG. 1 is a perspective view showing a configuration of a diaphragm-type pressure measuring chamber according to embodiment 1.
FIG. 2 is a vertical cross-sectional view of the pressure measuring chamber according to embodiment 1.
FIG. 3A is a perspective view showing the appearance of a diaphragm, and FIG. 3B is a cross-sectional view of the diaphragm.
FIG. 4A is an enlarged cross-sectional view of a part, of the diaphragm, enclosed by the imaginary circle IVA in FIG. 3B, and FIG. 4B is an enlarged cross-sectional view of a part, of the pressure measuring chamber, enclosed by the imaginary circle IVB in FIG. 2.
FIG. 5 is a perspective view showing a configuration of a diaphragm-type pressure measuring chamber according to embodiment 2.
FIG. 6 is a vertical cross-sectional view of the pressure measuring chamber according to embodiment 2.
FIG. 7 is a cross-sectional view of a diaphragm.
FIG. 8A is an enlarged cross-sectional view of a part, of the diaphragm, enclosed by the imaginary circle VIIIA in FIG. 7, and FIG. 8B is an enlarged cross-sectional view of a part, of the pressure measuring chamber, enclosed by the imaginary circle VIIIB in FIG. 6.
FIG. 9 is a cross-sectional view of a diaphragm according to modification 1.
FIG. 10A is an enlarged cross-sectional view of a part of the diaphragm according to modification 1, and FIG. 10B is an enlarged cross-sectional view of a part of a pressure measuring chamber mounted with the diaphragm according to modification 1.
FIG. 11 is an enlarged cross-sectional view of a part of a pressure measuring chamber mounted with a diaphragm according to modification 2.
FIG. 12 is an enlarged cross-sectional view of a part of a pressure measuring chamber mounted with a diaphragm according to modification 3.
FIG. 13 is a cross-sectional view of a diaphragm according to modification 4.
FIG. 14A is an enlarged cross-sectional view of a part of the diaphragm according to modification 4, and FIG. 14B is an enlarged cross-sectional view of a part of a pressure measuring chamber mounted with the diaphragm according to modification 4.
FIG. 15A is a plan view showing the appearance of a configuration of a pressure measuring chamber, FIG. 15B is a cross-sectional view taken along the line B-B in FIG. 15A, and FIG. 15C is an enlarged view which is a part of the cross-sectional view in FIG. 15B.
FIG. 16A is a perspective view of a diaphragm according to embodiment 3, FIG. 16B is a cross-sectional view, taken along the line B-B, of the diaphragm in FIG. 16A, and FIG. 16C is an enlarged view which is a part of the cross-sectional view in FIG. 16B.
FIG. 17 is a schematic diagram showing an example of a molding process for the diaphragm.
FIG. 18A to FIG. 18D are perspective views of the appearances of respective different modifications of a flexible membrane.

### Description of Embodiments

Hereinafter, diaphragm-type pressure measuring chambers according to embodiments of the present disclosure will be described with reference to the drawings. Directions used in the following description are conceptually used for the sake of convenience in explanations and do not limit directions or the like of each of the disclosed constituents to these directions. Also, the pressure measuring chambers described below are merely embodiments of the present disclosure. Therefore, the present disclosure is not limited to the following embodiments, and constituents may be added, deleted, or changed without departing from the gist of the disclosure.

### (Embodiment 1)

A pressure measuring chamber 1 according to the present disclosure forms a part of a blood circuit provided to a dialysis system and measures a pressure of blood of a patient flowing through blood flow paths provided to the blood circuit. The blood circuit includes, for example: a blood removal line as a blood flow path for guiding blood of a patient to the outside of the body; a filter which is connected to the blood removal line and through which a specific component is transferred from the blood of the patient to a dialysate to purify the blood; and a blood returning line as a blood flow path for returning the purified blood to the patient. A blood pump for causing the blood to flow through the blood circuit is also provided halfway through the blood removal line.

The dialysis system includes a pressure measuring device in addition to the blood circuit. The pressure measuring device includes a plurality of pressure sensors in the device, and each of the pressure sensors is connected to a corresponding place (pressure measuring place) in the blood circuit via a pressure monitoring line. The pressure measuring chamber 1 according to the present disclosure is provided between the pressure monitoring line and the pressure measuring place in the blood circuit.

The pressure measuring chamber 1 may be provided halfway through a blood flow line in the blood circuit so as to form a part of the line. Alternatively, the pressure measuring chamber 1 may be provided to be connected to another line having branched off from the blood flow line in the blood circuit via a drip chamber or the like. In the former case, blood passes through the inside (first chamber 13a described later) of the pressure measuring chamber 1. In the latter case, blood basically does not pass through the inside of the pressure measuring chamber 1, and air in contact with the blood in the blood circuit passes through the inside of the pressure measuring chamber 1.

### [Pressure Measuring Chamber]

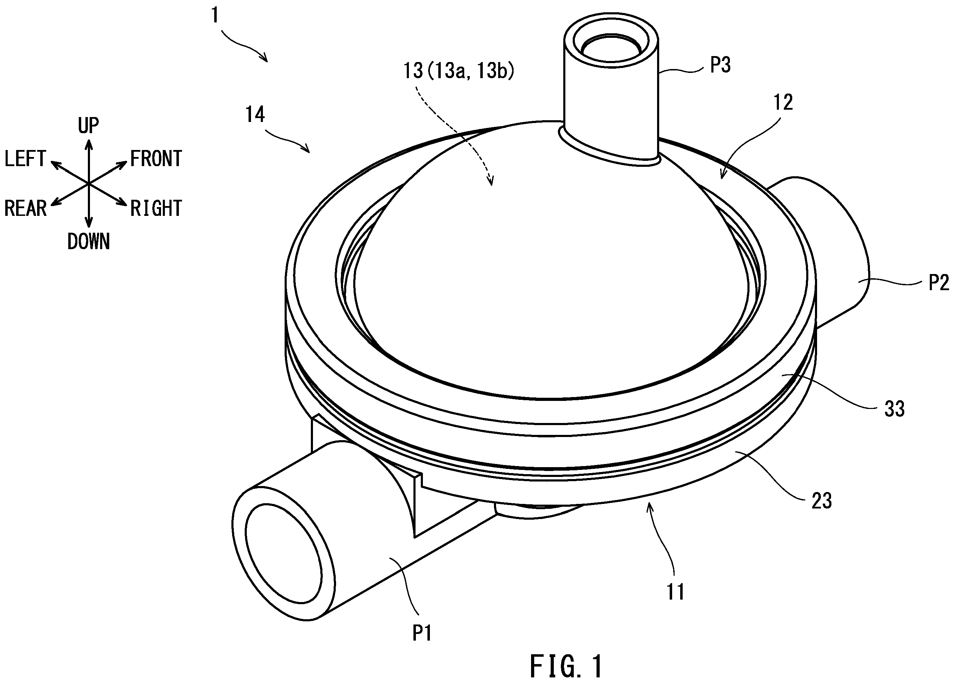

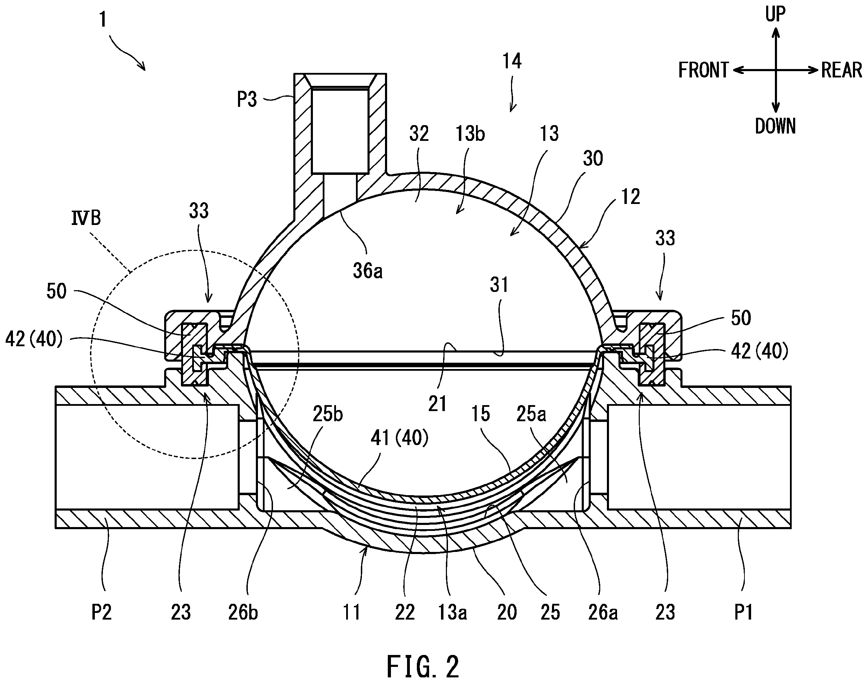

Hereinafter, a configuration of the pressure measuring chamber 1 will be described in detail. FIG. 1 is a perspective view showing a configuration of a diaphragm-type pressure measuring chamber 1 according to the present embodiment 1. FIG. 2 is a vertical cross-sectional view of the pressure measuring chamber 1.

The pressure measuring chamber 1 has a housing 14 in which a chamber space 13 is formed by combining a first case 11 and a second case 12 each formed from resin. Inside the housing 14, a flexible diaphragm 15 is provided and partitions the chamber space 13 into a first chamber 13a on the first case 11 side and a second chamber 13b on the second case 12 side. That is, both the first chamber 13a and the second chamber 13b are in contact with the diaphragm 15 and are separated from each other by the diaphragm 15 in an airtight and liquid-tight manner. A first port P1 and a second port P2 are formed in the first case 11, and a third port P3 is formed in the second case 12.

The external shape of this pressure measuring chamber 1 is substantially the shape of a circular dome, and the orientation thereof during use is not particularly limited. However, directions regarding the pressure measuring chamber 1 are defined as follows for convenience in explanations. That is, a direction in which the second case 12 is located relative to the first case 11 is defined as "upward direction", and the direction opposite to the upward direction is defined as "downward direction". Also, a direction in which the second port P2 is located relative to the first port P1 is defined as "frontward direction", and the direction opposite to the frontward direction is defined as "rearward direction". Furthermore, a direction intersecting with both the upward/downward direction and the frontward/rearward direction is defined as "leftward/rightward direction" as seen in the frontward direction.

Although a dome-shaped pressure measuring chamber 1 having a circular shape in a plan view (as seen from above) and bulged upward in an arc shape in a side view as shown in FIG. 1 will be described as an example in the present embodiment, the configuration of the pressure measuring chamber 1 is not limited thereto. For example, the pressure measuring chamber may have an elliptical shape in the plan view and be bulged upward in an arc shape in the side view, i.e., may have an oval shape. Alternatively, another shape may be employed. The positions at which the respective ports are attached to the housing are not particularly limited, either.

### [First Case]

The first case 11 includes a case body 20, and the first port P1 and the second port P2 are connected to the case body 20. The case body 20 has an opening 21 opened upward and has an inner surface 22 dented downward. The inner surface 22 and the lower surface of the diaphragm 15 form the first chamber (blood-side space) 13a which is a lower space of the chamber space 13. That is, the first chamber 13a is a space formed between the first case 11 and the diaphragm 15 and in communication with a blood flow path in the blood circuit. A shape of the chamber space 13 as seen in a plan view along the upward/downward direction (i.e., the shape of the opening 21) is substantially a perfect circle shape. However, the shape of the chamber space 13 is not limited thereto. For example, the shape as seen in the plan view may be an elliptical shape or another shape. Shapes of the chamber space 13 as seen in the frontward/rearward direction and the leftward/rightward direction are not particularly limited, either. For example, perfect circle shapes, elliptical shapes, rectangular shapes, or other shapes may be employed as appropriate.

The opening 21 of the case body 20 is provided with a first circling portion 23 which spreads radially outward from the upper end of the inner surface 22 and which is formed to circle the opening 21. The first circling portion 23 sandwiches and fixes (described later in detail) the diaphragm 15 between the first circling portion 23 and a second circling portion 33 of the second case 12 described later.

The case body 20 has a rear portion with which the first port P1 is integrated. The first port P1 has a cylindrical shape and has therein a flow path which is in communication with the first chamber 13a inside the case body 20. The case body 20 has a front portion with which the second port P2 is integrated. The second port P2 has a cylindrical shape and has therein a flow path which is also in communication with the first chamber 13a. The flow paths of these two first and second ports P1 and P2 are arranged to be coaxial with each other and have center lines extending in the frontward/rearward direction. The blood flow path provided to the above blood circuit is connected to the first port P1 and the second port P2. Blood having flowed from the upstream side of the blood flow path flows into the first chamber 13a via the first port P1 and then flows out to the downstream side of the blood flow path via the second port P2.

The inner surface 22 of the case body 20 has a bottom portion provided with a groove 25 extending along the frontward/rearward direction and formed downward from the inner surface 22. The groove 25 extends from a position at which the first port P1 is connected to the case body 20 to a position at which the second port P2 is connected to the case body 20. Therefore, a space 25a as a part of the rear side of the groove 25 is present between the inner surface 22 and a hole 26a on a side, of the first port P1, that is in communication with the first chamber 13a. That is, the hole 26a of the first port P1 is formed downward from the inner surface 22 of the case body 20 by the depth of the space 25a. Likewise, a space 25b formed by the groove 25 is present between the inner surface 22 and a hole 26b on a side, of the second port P2 on the front side, that is in communication with the first chamber 13a.

Such a groove 25 prevents the hole 26a of the first port P1 from being closed by the diaphragm 15 described later even when, for example, the internal pressure of the first chamber 13a decreases so that the diaphragm 15 significantly warps downward. In addition, a pressure transmitted through the first port P1 into the case body 20 acts on a wide region of the lower surface of the diaphragm 15 via spaces (spaces through which the ports P1 and P2 and the first chamber 13a are in communication with each other) such as the spaces 25a and 25b formed by the groove 25. Therefore, even in such a situation, the pressure measuring chamber 1 can accurately measure the pressure.

The portion that exhibits the above advantageous effects is not limited to the groove 25. For example, a projecting rib extending in the frontward/rearward direction may be formed on the inner surface 22 instead of or in addition to the groove 25. Alternatively, a plurality of protrusions may be formed on the inner surface 22 instead of or in addition to the groove 25. That is, the contact area between the diaphragm 15 and the inner surface 22 (in particular, a portion near the hole 26a) of the case body 20 only has to be decreased, and a recess or a protrusion may be provided to the inner surface 22 in order to form a space through which the first chamber 13a and the first port P1 are in communication with each other.

### [Second Case]

The second case 12 includes a case body 30, and the third port P3 is connected to the case body 30. The case body 30 substantially has the shape of a dome bulged upward, has an opening 31 opened downward, and has an inner surface 32 dented upward. The inner surface 32 and the upper surface of the diaphragm 15 form the second chamber (sensor-side space) 13b as an upper space of the chamber space 13.

The opening 31 of the case body 30 is provided with a second circling portion 33 which spreads radially outward from the lower end of the inner surface 32 and which is formed to circle the opening 31. The second circling portion 33 sandwiches (described later in detail) the diaphragm 15 between the second circling portion 33 and the above first circling portion 23 of the first case 11.

The case body 30 has a front portion with which the third port P3 is integrated. The pressure monitoring line extending from the above pressure measuring device is connected to the third port P3. The third port P3 has a cylindrical shape and extends upward from the outer surface of the case body 30 such that the center line of the third port P3 extends in the upward/downward direction. The third port P3 has an internal passage having a lower-end hole 36a through which the internal passage is in communication with the second chamber 13b inside the case body 30. On the inner surface 32 of the case body 30, for example, a plurality of linear ribs are formed in order to form an air flow path between the inner surface 32 and the diaphragm 15.

### [Diaphragm]

The diaphragm 15 has a flexible membrane 40 deformable in a first direction according to a pressure difference between the first chamber 13a and the second chamber 13b and a support frame 50 provided on a peripheral portion of the flexible membrane 40 and fixed between a first connection portion 60 and a second connection portion 70 described later. Here, the flexible membrane 40 has a surface area larger than a cross-sectional area of the chamber space 13 and has a three-dimensional shape at least partially protruding in the first direction. The support frame 50 has a higher rigidity than the flexible membrane 40. Consequently, ease in handling during assembly is realized. More detailed description will be given below.

The diaphragm 15 is a dome-shaped membrane having moderate flexibility and is deformable in the upward/downward direction (first direction) toward the first chamber 13a or the second chamber 13b according to the pressure difference between the first chamber 13a and the second chamber 13b (the difference between the respective internal pressures). That is, a configuration is employed in which the direction (upward/downward direction) in which the diaphragm 15 is deformable owing to a change in the pressure difference between the first chamber 13a and the second chamber 13b intersects with the direction (frontward/rearward direction) in which a fluid passes in the port P1 of the first chamber. The diaphragm 15 has the flexible membrane 40 and the support frame 50 which has an annular shape and which is provided on the peripheral portion of the flexible membrane 40. The flexible membrane 40 has a dome-shaped membrane portion 41 and an annular flange portion 42 extending outward from a peripheral edge of the membrane portion 41. The support frame 50 is connected to the flange portion 42.

Although a hemispherical diaphragm has been presented as an example of the dome-shaped diaphragm 15 in the present embodiment, the shape is not limited to a hemispherical shape. That is, the dome-shaped diaphragm in the present disclosure may have a shape with a rectangular cross section, the shape of one of the pieces obtained by horizontally splitting a torus (in other words, by bisecting the torus along a plane orthogonal to a center line passing through the center hole), or the like. Also, the shape of the diaphragm 15 is not limited to a dome shape, and another shape may be employed. For example, a flexible membrane having a spherical wave shape with waves being concentrically located as seen in the plan view may be employed. For the diaphragm 15, a configuration in which H/W as the ratio of a height dimension H to a width dimension W of the membrane portion 41 is not less than 0.1 and not more than 0.6 may be preferably employed, and a configuration in which H/W is not less than 0.2 and not more than 0.5 may be more preferably employed. Consequently, the positional shift amount of the membrane portion 41 according to a change in pressure can be increased without excessively increasing the dimension in the height direction, and the accuracy of pressure detection can be increased. Regarding the dimensions W and H, with the membrane portion 41 protruding to the second chamber 13b side, the maximum opening diameter on the first chamber 13a side is defined as the width dimension W, and the maximum height on the first chamber 13a side is defined as the height dimension H. The same applies to other embodiments and modifications.

### [Assembling Method for Pressure Measuring Chamber]

The diaphragm 15 and the housing 14 composed of the first case 11 and the second case 12 described above are combined, whereby the pressure measuring chamber 1 is formed. Specifically, first, the diaphragm 15 in such an orientation as to be dented downward (such an orientation as to opened upward) is mounted from above onto the first case 11 with the opening 21 thereof being oriented upward, for example. At this time, lower surfaces of the flange portion 42 and the support frame 50 of the diaphragm 15 are located to be opposed to the first circling portion 23 of the first case 11. Then, the first circling portion 23 and the support frame 50 are connected to each other (through, for example, adhesion or ultrasonic welding).

Next, the second case 12 with the opening 31 thereof being oriented downward is mounted from above onto the first case 11 in this state. At this time, the second circling portion 33 of the second case 12 is located to be opposed to the flange portion 42 and the support frame 50 of the diaphragm 15. Then, the second circling portion 33 and the support frame 50 are connected to each other (through, for example, adhesion or ultrasonic welding).

In this manner, the first case 11 and the second case 12 are adhered or welded to each other with the support frame 50 being sandwiched between peripheral edge portions of the respective cases, and the diaphragm 15 is supported between the first case 11 and the second case 12. As a result, the chamber space 13 is formed inside the housing 14, and the diaphragm 15 partitions the chamber space 13 into the first chamber 13a in communication with the first port P1 and the second port P2 and the second chamber 13b in communication with the third port P3.

The above connection methods between these constituents are merely examples, and no limitation thereto is made. For example, the connection method between the first circling portion 23 and the support frame 50 and the connection method between the second circling portion 33 and the support frame 50 are not limited to adhesion or welding, and these connections may be achieved through physical fitting that involves engagements between these constituents with recesses and protrusions, for example. Alternatively, with gaps being provided, connections (engagements) between these constituents do not have to be made. Likewise, the connection method between the first case 11 and the second case 12 is not limited to adhesion or welding, either, and this connection may also be achieved through physical fitting that involves engagement between these constituents with recesses and protrusions, for example.

As described above, the pressure measuring chamber 1 may be provided halfway through a blood flow line (blood flow path) in the blood circuit. In this case, the first port P1 serves as an inlet for the blood, the second port P2 serves as an outlet for the blood, and the blood passes through the first chamber 13a. Alternatively, the pressure measuring chamber 1 may be connected to another line (blood flow path) having branched off from the blood flow line in the blood circuit via a drip chamber. In this case, the first port P1 serves as a connection port to the downstream end of the other line, the second port P2 is closed, and the first chamber 13a is in communication with a space occupied by air in contact with blood in the drip chamber. In either case, the third port P3 is connected to one end of the pressure monitoring line, and another end of the pressure monitoring line is connected to the pressure sensor of the pressure measuring device. Therefore, the pressure measuring device measures a pressure in the second chamber 13b via the third port P3. In this manner, the first chamber 13a serves as a blood-side space, and the second chamber 13b serves as a sensor-side space.

### [Structure regarding Ease of Assembly]

Here, as the flexibility of the diaphragm 15 becomes higher, responsiveness thereof to a pressure (blood pressure) in the first chamber 13a becomes better, and the accuracy of pressure measurement is further improved. Meanwhile, as the flexibility of the material selected for the diaphragm 15 becomes higher, the shape stability (shape retainability) thereof becomes lower, whereby workability during assembly becomes lower. In view of this, the diaphragm 15 of the pressure measuring chamber 1 according to the present disclosure is composed of the flexible membrane 40 formed from a highly flexible material and the support frame 50 formed from a material having a higher rigidity than the flexible membrane 40, whereby improvement of ease of assembly and the like are achieved. Hereinafter, such a structure regarding ease of assembly will be described.

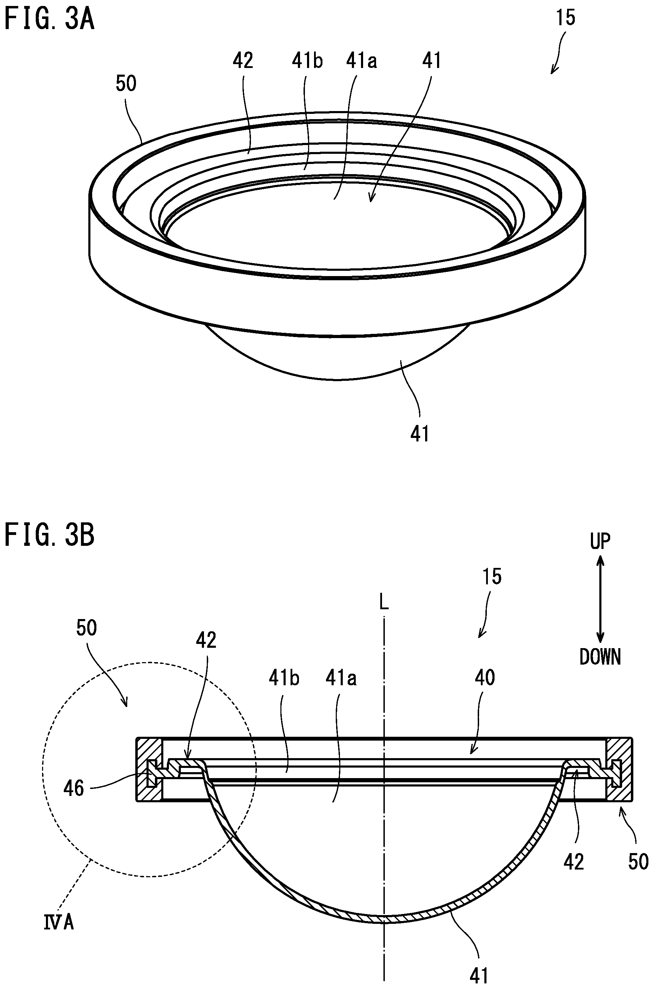

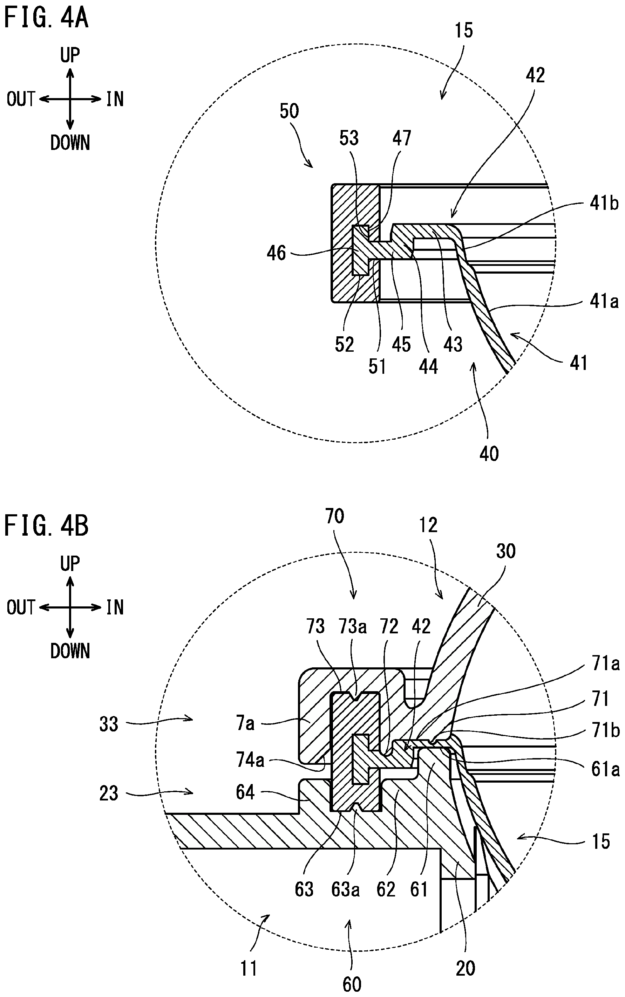

FIG. 3A is a perspective view showing the appearance of the diaphragm 15, and FIG. 3B is a cross-sectional view of the diaphragm 15. FIG. 4A is an enlarged cross-sectional view of a part, of the diaphragm 15, enclosed by the imaginary circle IVA in FIG. 3B, and FIG. 4B is an enlarged cross-sectional view of a part, of the pressure measuring chamber 1, enclosed by the imaginary circle IVB in FIG. 2.

As shown in FIG. 3A and FIG. 3B, the diaphragm 15 includes the flexible membrane 40 and the support frame 50, has a dome shape on the whole, and has a structure rotationally symmetric about a predetermined imaginary line L extending in the upward/downward direction. The imaginary line L is a line passing through the center of the dome-shaped membrane portion 41. Hereinafter, the "upward/downward direction" used in explanations made with reference to FIG. 3A, FIG. 3B, FIG. 4A, and FIG. 4B is identical to the "upward/downward direction" used in the explanations made with reference to FIG. 1 and FIG. 2. Meanwhile, since the diaphragm 15 is rotationally symmetric about the imaginary line L as described above, a direction toward the imaginary line L is referred to as "inward direction" or "inner side", and a direction away from the imaginary line L is referred to as "outward direction" or "outer side". Furthermore, a direction orthogonal to the upward/downward direction is referred to as "horizontal direction", and the horizontal direction is identical to the inward/outward direction.

As shown in FIG. 3A and FIG. 3B, the flexible membrane 40 of the diaphragm 15 has the dome-shaped membrane portion 41, and the membrane portion 41 is deformable in the upward/downward direction according to the pressure difference between the first chamber 13a and the second chamber 13b. The membrane portion 41 has: a body portion 41a having a predetermined thickness dimension and having a dome shape; and a small-thickness portion 41b having a smaller thickness dimension than the body portion 41a and extending upward from a peripheral edge, i.e., opening end, of the body portion 41a. The small-thickness portion 41b has the shape of a belt having a predetermined width dimension and has an annular shape circling along the opening end of the body portion 41a. The body portion 41a is connected to one end (in FIG. 3B, the lower end) in the width direction of the small-thickness portion 41b, and the flange portion 42 is connected to another end (in FIG. 3B, the upper end) in the width direction of the small-thickness portion 41b. The thickness of the body portion 41a of the membrane portion 41 can be set as appropriate to fall within a range of, for example, not less than 0.2 mm and not more than 1.5 mm. The small-thickness portion 41b is formed to have an even smaller thickness than the body portion 41a, whereby the membrane portion 41 composed of the body portion 41a and the small-thickness portion 41b has such a configuration as to not easily undergo elastic restoration upon deformation, i.e., such a configuration as to easily retain the post-deformation shape. The same applies to the other embodiments and modifications described below.

The diaphragm 15 has a surface area larger than a cross-sectional area of the chamber space 13. For example, a cross-sectional area obtained by cutting the chamber space 13 along a joint plane between the first case 11 and the second case 12 is approximately equal to the area of the opening 21 of the first case 11. Meanwhile, the membrane portion 41 of the diaphragm 15 closes the entire opening of the first case 11 and has a dome shape as described above. Thus, the membrane portion 41 has a surface area larger than the area of the opening 21 of the first case 11 and hence larger than the cross-sectional area of the chamber space 13.

As shown in FIG. 4A, the flange portion 42 has a first portion (seal portion) 43 extending outward from the other end of the small-thickness portion 41b. The first portion 43 has a substantially horizontal plate shape and circles along the small-thickness portion 41b to have an annular shape. The first portion 43 has an outer end to which the upper end of a second portion 44 extending in the upward/downward direction is connected. The second portion 44 also circles along the first portion 43 to have an annular shape. The second portion 44 has a lower end to which a third portion 45 extending outward is connected. The third portion 45 also circles along the second portion 44 to have an annular shape. As shown in FIG. 4A, the first portion 43, the second portion 44, and the third portion 45 form a crank-like cross-sectional shape.

The third portion 45 has an outer end provided with an enlarged portion 46. In an example, the enlarged portion 46 has a vertically-elongated rectangular cross-sectional shape with the dimension thereof in the upward/downward direction being larger than the dimension thereof in the horizontal direction, and the dimension in the upward/downward direction of the enlarged portion 46 is larger than the dimension in the upward/downward direction of the third portion 45. That is, the enlarged portion 46 has such a configuration as to extend upward and downward from the outer end of the third portion 45, and a structure obtained by connecting the third portion and the enlarged portion 46 has a shape obtained by tilting a T shape by 90°.

Meanwhile, the support frame 50 has an annular shape and has a vertically-elongated rectangular cross-sectional shape with the width dimension thereof in the upward/downward direction being larger than the thickness dimension thereof in the horizontal direction. More specifically, the support frame 50 has a cross-sectional shape with the dimension thereof in the upward/downward direction being larger than the dimension in the upward/downward direction of the above enlarged portion 46 and with the dimension thereof in the horizontal direction also being larger than the dimension in the horizontal direction of the enlarged portion 46. An outer part of the third portion 45 and the enlarged portion 46 are embedded in such a support frame 50. The dimension in the upward/downward direction of the support frame 50 is smaller than the overall height dimension of the dome-shaped membrane portion 41 of the flexible membrane 40 (the same applies to support frames 50A to 50D described later).

Specifically, the support frame 50 has a first space 51 opened in the inner peripheral surface thereof and a second space 52 formed on the farther side (outer side) relative to the first space 51. The second space 52 has a larger dimension in the upward/downward direction than the first space 51, and a combination of the first space 51 and the second space 52 has a cross-sectional shape obtained by tilting a T shape by 90°. The outer part of the third portion 45 is embedded in the first space 51, and the enlarged portion 46 is embedded in the second space 52.

In such a configuration, the flexible membrane 40 and the support frame 50 are partially opposed to and in contact with each other in the horizontal direction (inward/outward direction), whereby the flexible membrane 40 is prevented from coming off from the support frame 50. Specifically, as shown in FIG. 4A, the support frame 50 has a wall surface delimiting the second space 52, and the wall surface has an engaging surface 53 oriented outward. The engaging surface 53 is provided at each of two positions which are positions above and below the first space 51. Meanwhile, the enlarged portion 46 located at the peripheral portion of the flexible membrane 40 has a to-be-engaged surface 47 oriented inward. The to-be-engaged surface 47 is provided at each of two positions which are positions above and below the third portion 45.

Each of the engaging surfaces 53 and the corresponding to-be-engaged surface 47 are opposed to and in contact with each other in the horizontal direction. Therefore, when the flexible membrane 40 is deformed according to the pressure difference so that the flange portion 42 is pulled inward during pressure measurement, the to-be-engaged surface 47 of the flexible membrane 40 is engaged with the engaging surface 53 of the support frame 50. Therefore, the flexible membrane 40 is prevented from coming off from the support frame 50.

The flexible membrane 40 of the diaphragm 15 such as one described above is formed from a highly flexible material. For example, a material such as a synthetic rubber such as isoprene rubber or silicone rubber, natural rubber, soft polyvinyl chloride, or thermoplastic elastomer may be used. With materials suitable for the flexible membrane 40 being considered from the viewpoint of physical properties, a material having a Shore A hardness of not less than 10 and less than 80 and more preferably not less than 20 and not more than 60 is considered to be suitable, for example. Meanwhile, the support frame 50 of the diaphragm 15 is formed from a material having a higher rigidity than the flexible membrane 40. For example, a material such as polyethylene, polycarbonate, polypropylene, hard polyvinyl chloride, or polyester may be used. Alternatively, metal, ceramic, or the like may also be used instead of such hard resins. Likewise, with materials suitable for the support frame 50 being considered from the viewpoint of physical properties, a material having a Shore A hardness of not less than 80 and more preferably not less than 90 is considered to be suitable, for example.

The diaphragm 15 in which the flexible membrane 40 and the support frame 50 are formed from different materials as described above can be molded by performing two-shot molding or insert molding on the flexible membrane 40 and the support frame 50 in a mutually engaged state. For example, in a case where both the flexible membrane 40 and the support frame 50 are formed from resin, either of two-shot molding and insert molding may be performed, and, in a case where the support frame 50 is formed from metal or ceramic, insert molding may be suitably selected.

As shown in FIG. 4B, the first circling portion 23 of the first case 11 and the second circling portion 33 of the second case 12 are respectively provided with a first connection portion 60 and a second connection portion 70 in order to sandwich the above diaphragm 15.

The first connection portion 60 has a first portion 61 located on the opening end of the case body 20. The first portion 61 has a first surface 61a which faces upward. The first surface 61a is a horizontal surface orthogonal to the upward/downward direction. On the outer side relative to the first portion 61, a second portion 62 dented downward is provided, and, on the further outer side relative to the second portion 62, a first insertion groove 63 dented further downward is provided. In addition, on the outer side relative to the first insertion groove 63, an outer peripheral wall 64 extending upward is provided.

The second connection portion 70 has a first portion 71 extending outward from the opening end of the case body 30. The first portion 71 has a first surface 71a which is a horizontal surface orthogonal to the upward/downward direction and which faces downward. The first surface 71a has a larger dimension in the horizontal direction than the first surface 61a of the first case 11. Specifically, the inner end of the first surface 61a and the inner end of the first surface 71a are substantially at the same position, but the outer end of the first surface 71a is located on the outer side relative to the outer end of the first surface 61a.

On the outer side relative to the first portion 71, a second portion 72 protruding downward is provided. The second portion 72 is opposed to and located above an outer part of the second portion 62 of the first case 11. On the outer side relative to the second portion 72, a second insertion groove 73 dented upward is provided. The second insertion groove 73 is opposed to and located above the first insertion groove 63 of the first case 11. Furthermore, on the outer side relative to the second insertion groove 73, an outer peripheral wall 74 extending downward is provided. The outer peripheral wall 74 is opposed to and located above the outer peripheral wall 64 of the first case 11.

A crank-shaped space is formed between: the first portion 61 and the second portion 62 of the first connection portion 60; and the first portion 71 and the second connection portion 72 of the second connection portion 70. The first portion 43, the second portion 44, and the third portion 45 which belong to the flange portion 42 of the flexible membrane 40 and which form a crank-shaped portion are sandwiched in this crank-shaped space.

That is, the first portion (seal portion) 43 of the flexible membrane 40 is sandwiched and compressed between the first portion 61 of the first connection portion 60 and an inner part of the first portion 71 of the second connection portion 70. In addition, the second portion 44 of the flexible membrane 40 is sandwiched between an inner part of the second portion 62 of the first connection portion 60 and an outer part of the first portion 71 of the second connection portion 70. Furthermore, the third portion 45 of the flexible membrane 40 is sandwiched between the outer part of the second portion 62 of the first connection portion 60 and the second portion 72 of the second connection portion 70.

Consequently, improvement of the positioning accuracy for the diaphragm 15 with respect to the first case 11 and the second case 12 is achieved. In addition, the first portion 71 of the second connection portion 70 is provided with a rib 71b protruding downward from the first surface 71a toward the first surface 61a of the first connection portion 60. Therefore, the rib 71b bites into the upper surface of the first portion 43 of the flexible membrane 40, whereby sealability is ensured.

In addition, the first insertion groove 63 of the first connection portion 60 and the second insertion groove 73 of the second connection portion 70 are located to be opposed to each other in the upward/downward direction and form a vertically-elongated space in the upward/downward direction. The support frame 50 is sandwiched in the vertically-elongated space. That is, the support frame 50 is fitted downward into the first insertion groove 63 and is fitted upward into the second insertion groove 73. The first case 11 and the diaphragm 15 are connected to each other by being subjected to ultrasonic welding or the like between the bottom surface of the first insertion groove 63 and the lower surface of the support frame 50. In addition, the second case 12 and the diaphragm 15 are connected to each other by being subjected to ultrasonic welding or the like between the bottom surface (ceiling surface) of the second insertion groove 73 and the upper surface of the support frame 50.

As shown in FIG. 4B, the bottom surface of the first insertion groove 63 is provided with a rib 63a protruding upward, and the rib 63a is pressure-bonded to the support frame 50 upon connection between the first case 11 and the diaphragm 15. Likewise, the bottom surface (ceiling surface) of the second insertion groove 73 is provided with a rib 73a protruding downward, and the rib 73a is pressure-bonded to the support frame 50 upon connection between the second case 12 and the diaphragm 15. Therefore, sealability at each of the connection point between the first case 11 and the diaphragm 15 and the connection point between the second case 12 and the diaphragm 15 is ensured. Ultrasonic welding may be performed between the bottom surface of the first insertion groove 63 and the lower surface of the support frame 50 and/or between the bottom surface (ceiling surface) of the second insertion groove 73 and the upper surface of the support frame 50. In this case, the ribs 63a and 73a serve as protrusions for welding and are melted through the ultrasonic welding to form welded surfaces (in this case, the ribs 63a and 73a in FIG. 4B in the forms of triangular constituents disappear after the welding).

In addition, a tapered surface 74a is formed at the opening edge of the second insertion groove 73 of the second case 12, i.e., at the connection point between the lower surface and the inner surface of the outer peripheral wall 74 delimiting the second insertion groove 73 in the example in FIG. 4B. The tapered surface 74a is a surface tilted such that a portion thereof on a further lower side is located on a further outer side. The tapered surface 74a allows the opening area of the second insertion groove 73 to increase in the downward direction. Therefore, workability in inserting the support frame 50 into the second insertion groove 73 is favorable.

The pressure measuring chamber 1 having the above configuration is assembled as follows. First, a lower portion of the support frame 50 of the diaphragm 15 is fitted into the first insertion groove 63 of the first case 11. At this time, the first portion 43 of the flexible membrane 40 of the diaphragm 15 is placed on the first surface 61a which is the upper surface of the first portion 61 of the first case 11. Next, the second case 12 is placed. At this time, an upper portion of the support frame 50 is fitted into the second insertion groove 73 of the second case 12, and, as described above, the tapered surface 74a makes it easy to perform this fitting even when visibility is low during work.

In this manner, the diaphragm 15 is positioned with respect to the first case 11 and the second case 12 by the highly rigid support frame 50. Therefore, high positioning accuracy for the diaphragm 15 with respect to the first case 11 and the second case 12 can be ensured. Regarding this, description will be given as follows. The pressure measuring chamber 1 including the first case 11, the second case 12, and the flexible membrane 40 monitors blood pressure by measuring the pressure in the second chamber 13b. In this case, when the flexible membrane 40 generates a small reaction force in response to deformation and is easily deformable, the pressure in the first chamber 13a is directly usable as the pressure in the second chamber 13b, and thus such a flexible membrane 40 is preferable.

Therefore, the membrane portion 41 deformable according to the pressure in the flexible membrane 40 is preferably formed so as to have a surface area larger than the cross-sectional area of the pressure measuring chamber. Also, the membrane portion 41 is preferably formed so as not to be elastically deformed after deformation and so as to retain the post-deformation shape (so as not to restore the pre-deformation shape owing to elasticity). Also, the membrane portion 41 preferably has a small thickness so as to be easily deformed with a low pressure. However, as the reaction force to be generated in response to deformation of the membrane portion 41 of the flexible membrane 40 is made lower and the deformation is made easier, it becomes easier for the membrane portion 41 to be deformed owing to minute vibrations in the flexible membrane 40 alone. As a result, if the diaphragm 15 does not have the highly rigid support frame 50, the following drawback arises in a process for mounting the flexible membrane 40 onto the first case 11 or the second case 12. That is, when deformation of a part of the dome-shaped membrane portion 41 occurs, the deformation influences the peripheral edge of the flexible membrane 40, whereby the flexible membrane 40 may be positionally shifted from a position at which the flexible membrane 40 is assumed to be located with respect to the first case 11 and the second case 12.

When the positional shift is significant, stress is unevenly applied to the flexible membrane 40, and the dome-shaped membrane portion 41 is excessively pulled, whereby a wrinkle or a strain is generated. Such a wrinkle or strain generated at the time of fixing the flexible membrane 40 to the first case 11 and the second case 12 is not eliminated even when the dome-shaped membrane portion 41 is deformed according to the pressure. Consequently, this wrinkle or strain might cause an unintentional reaction force upon deformation of the membrane portion 41, might make it not easy for deformation to occur, or might otherwise influence the deformation performance of the membrane portion 41. Thus, this wrinkle or strain might influence the accuracy of pressure measurement.

However, the diaphragm 15 according to the present embodiment includes, on the peripheral edge thereof, the support frame 50 having a higher rigidity than the flexible membrane 40 as described above. Consequently, the peripheral edge, of the diaphragm 15, disposed inside an accommodation space 98 formed by the first case 11 and the second case 12 has a stable shape without being influenced by deformation of the membrane portion 41 and is less likely to be positionally shifted with respect to the first case 11 and the second case 12. Thus, the diaphragm 15 can be inhibited from sustaining a wrinkle or a strain when the diaphragm 15 is mounted onto the first case 11 and the second case 12.

The peripheral edge of the diaphragm 15 is sandwiched and compressed between the first case 11 and the second case 12, whereby the pressure measuring chamber 1 has liquid-tightness and airtightness. The liquid-tightness and the airtightness are preferably realized by sandwiching and compressing the flexible membrane 40 between the first case 11 and the second case 12. Alternatively, the liquid-tightness and the airtightness may be realized by sandwiching and compressing the support frame 50 between the first case 11 and the second case 12. The shape of the support frame 50 is not limited but is particularly preferably such a shape that the engaging surface 53 of the support frame 50 is located on the inner side relative to the to-be-engaged surface 47 of the flexible membrane 40, and the engaging surface 53 and the to-be-engaged surface 47 are opposed to each other in the inward/outward direction (see FIG. 4A). In this case, the area of the fixation portion between the support frame 50 and the flexible membrane 40 in an inner region of the peripheral edge of the flexible membrane 40 can be increased, and the to-be-engaged surface 47 of the flexible membrane 40 is opposed to the engaging surface 53 of the support frame 50 in the direction of coming-off from the support frame 50 (i.e., inward direction) so that the flexible membrane 40 can be effectively prevented from coming off, whereby positional shift of the diaphragm and occurrence of a wrinkle or a strain due to the positional shift can be further prevented.

Furthermore, when the support frame 50 is fitted into the second insertion groove 73, the first surface 71a of the second case 12 is opposed to the first surface 61a of the first case 11, and the first portion 43 of the flexible membrane 40 is sandwiched between the first surface 71a and the first surface 61a. At this time, the rib 71b protruding from the first surface 71a bites into the first portion 43. Therefore, the pressure measuring chamber 1 can ensure high sealability between the inside of the chamber space 13 and the outside of the housing 14.

As described above, the pressure measuring chamber 1 according to the present embodiment 1 is favorable in terms of ease of assembly and can be appropriately assembled. In addition, the flexible membrane 40 and the support frame 50 are formed from different materials, whereby respective materials suitable for the flexible membrane 40 and the support frame 50 can be separately selected from the viewpoint of performance (flexibility and rigidity) and dimension (thickness and the like). The above configuration is merely an example, and changes can be made as appropriate.

For example, although a configuration having the first insertion groove 63 and the second insertion groove 73 in order to achieve insertion into the support frame 50 has been presented as an example, a configuration may be employed in which only one of the insertion grooves is provided. Although the ribs 63a and 73a increase the sealability with respect to the support frame 50 as described above, the ribs 63a and 73a may be dispensed with. That is, it is allowable to provide only one of the ribs 63a and 73a or dispense with both of the ribs 63a and 73a. The first portion 43 of the flexible membrane 40 ensures sealability by allowing the rib 71b to bite into one surface thereof, and, in this case, a material having a higher rigidity than the flexible membrane 40 may be used for the other surface of the first portion 43. In a case where a sealing structure is formed with respect to the support frame 50 by the ribs 63a and 73a or the like as described above, a sealing structure formed by the first portion 43 of the flexible membrane 40 and the rib 71b may be dispensed with. Although a configuration in which the rib 71b is provided to the first surface 71a of the first portion 71 of the second case 12 has been presented as an example, a rib may be provided to the first surface 61a of the first portion 61 of the first case 11 instead of or in addition to the rib 71b.

Furthermore, the tapered surface 74a may also be dispensed with. In the case of providing the tapered surface, the tapered surface may be provided to the lower end of an inner wall surface of the second insertion groove 73, the upper end of an inner wall surface of the first insertion groove 63, or the upper end of an outer wall surface of the first insertion groove 63. Alternatively, the tapered surface may be provided to the connection point between the outer peripheral surface and the upper or lower surface of the support frame 50, the connection point between the inner peripheral surface and the upper or lower surface of the support frame 50, or the like.

### (Embodiment 2)

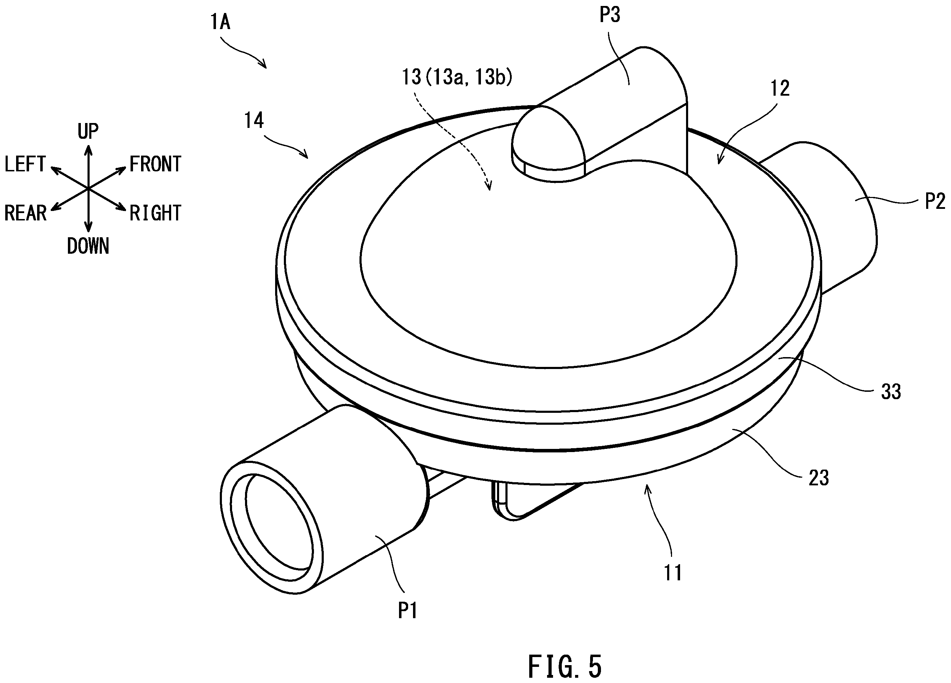

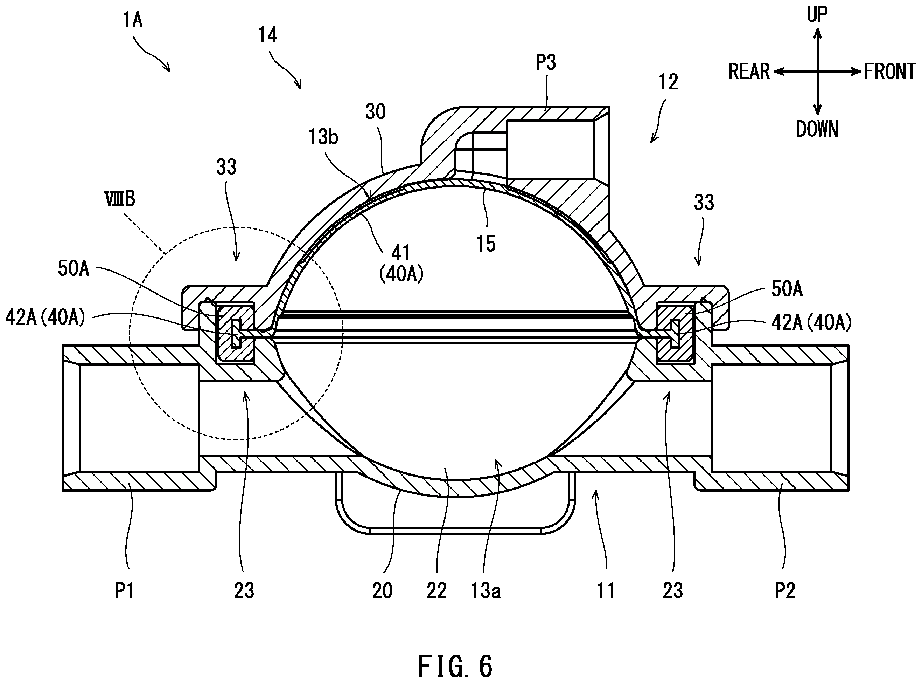

A configuration of a pressure measuring chamber 1A according to embodiment 2 of the present disclosure will be described. FIG. 5 is a perspective view showing the configuration of the pressure measuring chamber 1A. FIG. 6 is a vertical cross-sectional view of the pressure measuring chamber 1A. Hereinafter, constituents of the pressure measuring chamber 1A that differ from those of the pressure measuring chamber 1 according to embodiment 1 will be mainly described. Constituents of the pressure measuring chamber 1A that are similar to those of the pressure measuring chamber 1 are sometimes denoted by the same reference signs, and description thereof is sometimes omitted.

The pressure measuring chamber 1A has the housing 14 in which the chamber space 13 is formed by combining the first case 11 and the second case 12. Inside the housing 14, a flexible diaphragm 15 is provided and partitions the chamber space 13 into the first chamber 13a on the first case 11 side and the second chamber 13b on the second case 12 side. The cross-sectional view in FIG. 6 shows a configuration in which the diaphragm 15 is accommodated in a state of being bulged upward in a protruding shape.

The first port P1 and the second port P2 are formed in the first case 11, and a third port P3 having a configuration different from that of the third port P3 in embodiment 1 is formed in the second case 12. The third port P3 in embodiment 2 extends frontward from near the center in the frontward/rearward direction of an upper portion of the second case 12. Similar to the first port P1 and the second port P2, the third port P3 has an internal passage formed along the frontward/rearward direction. The internal passage has: a rear end opened downward near the top of the second case 12 (near the center in the frontward/rearward direction and in the leftward/rightward direction) and in communication with the second chamber 13b; and a front end opened frontward to the outside near a front portion of the second case 12.

Unlike the pressure measuring chamber 1, the pressure measuring chamber 1A is not provided with the groove 25 in the bottom portion of the first case 11. However, in the pressure measuring chamber 1A as well, the groove 25 may be provided in the bottom portion of the first case 11, or a protrusion may be provided on the inner surface 22 of the first case 11 instead of or in addition to the groove 25.

In the pressure measuring chamber 1A according to the present disclosure as well, the diaphragm 15 is composed of a flexible membrane 40A formed from a highly flexible material and a support frame 50A formed from a material having a higher rigidity than the flexible membrane 40A, whereby improvement of ease of assembly and the like are achieved. Hereinafter, such a structure regarding ease of assembly will be described.

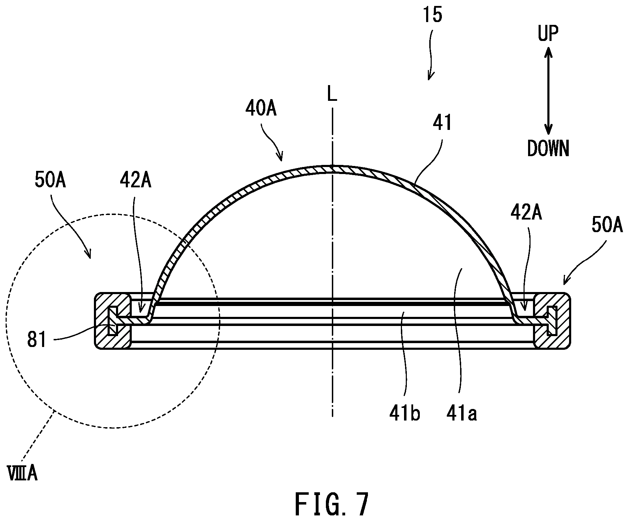

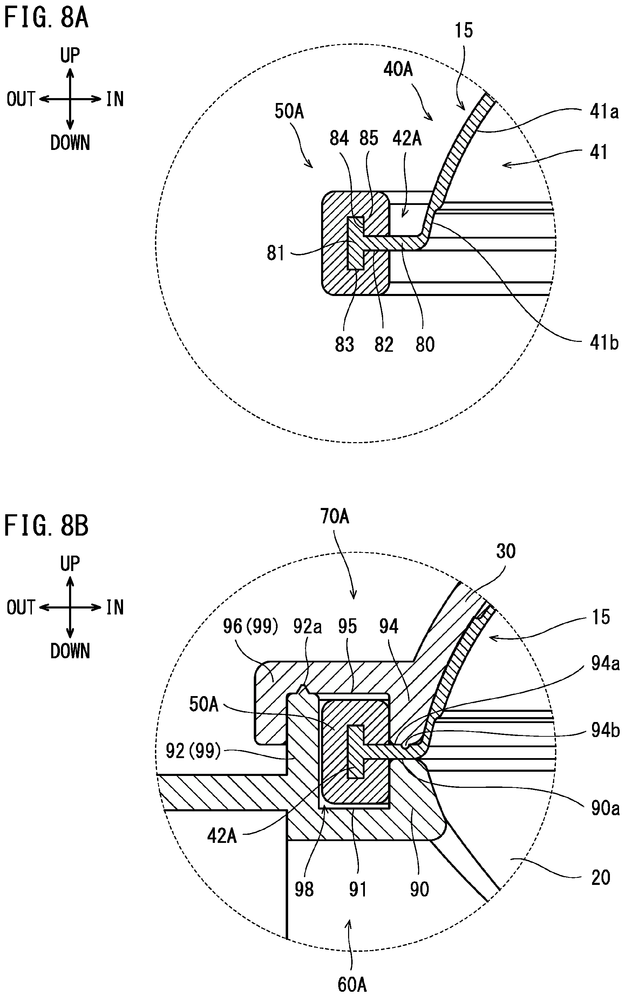

FIG. 7 is a cross-sectional view of the diaphragm 15. FIG. 8A is an enlarged cross-sectional view of a part, of the diaphragm 15, enclosed by the imaginary circle VIIIA in FIG. 7, and FIG. 8B is an enlarged cross-sectional view of a part, of the pressure measuring chamber 1A, enclosed by the imaginary circle VIIIB in FIG. 6.

As shown in FIG. 7, the diaphragm 15 includes the flexible membrane 40A and the support frame 50A, has a dome shape on the whole, and has a structure rotationally symmetric about the predetermined imaginary line L extending in the upward/downward direction. The flexible membrane 40A of the diaphragm 15 has: a membrane portion 41 composed of the dome-shaped body portion 41a and the small-thickness portion 41b; and a flange portion 42A. Out of these constituents, the membrane portion 41 is the same constituent as the membrane portion 41 according to embodiment 1. The above imaginary line L is a line passing through the center of the dome-shaped membrane portion 41.

The flange portion 42A has an externally-extending portion (seal portion) 80 extending outward from the other end of the small-thickness portion 41b. The externally-extending portion 80 has a substantially horizontal plate shape and circles along the small-thickness portion 41b to have an annular shape. The externally-extending portion 80 has an outer end provided with an enlarged portion 81. In an example, the enlarged portion 81 has a vertically-elongated rectangular cross-sectional shape with the dimension thereof in the upward/downward direction being larger than the dimension thereof in the horizontal direction, and the dimension in the upward/downward direction of the enlarged portion 81 is larger than the dimension in the upward/downward direction of the externally-extending portion 80. That is, the enlarged portion 81 has such a configuration as to extend upward and downward from the outer end of the externally-extending portion 80, and a structure obtained by connecting the externally-extending portion and the enlarged portion 81 has a shape obtained by tilting a T shape by 90°.

Meanwhile, the support frame 50A has an annular shape on the whole and has a vertically-elongated rectangular cross-sectional shape with the dimension thereof in the upward/downward direction being larger than the dimension thereof in the horizontal direction. More specifically, the support frame 50A has a cross-sectional shape with the dimension thereof in the upward/downward direction being larger than the dimension in the upward/downward direction of the above enlarged portion 81 and with the dimension thereof in the horizontal direction also being larger than the dimension in the horizontal direction of the enlarged portion 81. An outer part of the first portion 80 and the enlarged portion 81 are embedded in such a support frame 50A.

Specifically, the support frame 50A has a first space 82 opened in the inner peripheral surface thereof and a second space 83 formed on the farther side (outer side) relative to the first space 82. The second space 83 has a larger dimension in the upward/downward direction than the first space 82, and a combination of the first space 82 and the second space 83 has a cross-sectional shape obtained by tilting a T shape by 90°. The outer part of the first portion 80 is embedded in the first space 82, and the enlarged portion 81 is embedded in the second space 83.

As shown in FIG. 8A, the support frame 50A has a wall surface delimiting the second space 83, and the wall surface has an engaging surface 84 oriented outward. The engaging surface 84 is provided at each of two positions which are positions above and below the first space 82. Meanwhile, the enlarged portion 81 located at the peripheral portion of the flexible membrane 40A has a to-be-engaged surface 85 oriented inward. The to-be-engaged surface 85 is provided at each of two positions which are positions above and below the first portion 80.

Each of the engaging surfaces 84 and the corresponding to-be-engaged surface 85 are opposed to and in contact with each other in the horizontal direction. Therefore, when the flexible membrane 40A is deformed according to the pressure difference so that the flange portion 42A is pulled inward during pressure measurement, the to-be-engaged surface 85 of the flexible membrane 40A is engaged with the engaging surface 84 of the support frame 50A. Therefore, the flexible membrane 40A is prevented from coming off from the support frame 50A. In the diaphragm 15 in the present embodiment 2 as well, the materials forming the flexible membrane 40A and the support frame 50A are identical to those in the diaphragm 15 in embodiment 1.

As shown in FIG. 8B, the first circling portion 23 of the first case 11 and the second circling portion 33 of the second case 12 are respectively provided with a first connection portion 60A and a second connection portion 70A in order to sandwich and fix the above diaphragm 15.

The first connection portion 60A has: an inner peripheral wall 90 located at the opening end of the case body 20; an outer peripheral wall 92 located on the outer side relative to the inner peripheral wall 90; and a first insertion groove 91 located between the inner peripheral wall 90 and the outer peripheral wall 92. The inner peripheral wall 90 extends to protrude upward and has an upper end surface 90a which is a horizontal surface orthogonal to the upward/downward direction. The outer peripheral wall 92 extends to protrude upward and has an upper end located on the upper side relative to the upper end of the inner peripheral wall 90. The first insertion groove 91 as a space having a substantially rectangular cross-sectional shape is formed between the inner peripheral wall 90 and the outer peripheral wall 92. The first insertion groove 91 is a groove opened upward and is provided to circle along the opening of the case body 20. The upper end surface of the outer peripheral wall 92 is provided with a protrusion 92a which is for welding and which protrudes upward. The protrusion 92a is melted through welding to form a welded surface. The protrusion 92a shown in the drawing has a premelting protruding shape for easy understanding.

The second connection portion 70A has: an inner peripheral wall 94 located at the opening end of the case body 30; an outer peripheral wall 96 located on the outer side relative to the inner peripheral wall 94; and a second insertion groove 95 located between the inner peripheral wall 94 and the outer peripheral wall 96. The inner peripheral wall 94 extends to protrude downward and has a lower end surface 94a which is a horizontal surface orthogonal to the upward/downward direction and which is located to be opposed to the upper end surface 90a of the above inner peripheral wall 90. The lower end surface 94a is provided with a rib 94b protruding downward.

The outer peripheral wall 96 extends to protrude downward and has a lower end located at substantially the same height in the upward/downward direction as that of the lower end of the inner peripheral wall 94. The second insertion groove 95 as a space having a substantially rectangular cross-sectional shape is formed between the inner peripheral wall 94 and the outer peripheral wall 96. The second insertion groove 95 is a groove opened downward and is provided to circle along the opening of the case body 30. Here, the outer peripheral wall 92 of the first connection portion 60A and the outer peripheral wall 96 of the second connection portion 70A are each an opposed wall 99. These opposed walls 99 (outer peripheral wall 92 and outer peripheral wall 96) are opposed to each other in the inward/outward direction as shown in FIG. 8B. Consequently, positioning between the first case 11 and the second case 12 is facilitated.

Although a configuration in which the outer peripheral wall 96 of the second connection portion 70A is located on the outer side in the inward/outward direction relative to the outer peripheral wall 92 of the first connection portion 60A has been presented as an example in FIG. 8B, no limitation thereto is made. For example, a configuration may be employed in which the outer peripheral wall 96 of the second connection portion 70A is located on the inner side in the inward/outward direction relative to the outer peripheral wall 92 of the first connection portion 60A. The configuration of the opposed walls for positioning between the first case 11 and the second case 12 is not limited to the configuration of the above outer peripheral walls 92 and 96. In addition to or instead of the outer peripheral walls 92 and 96, opposed walls for mutual positioning may be provided at any positions between the first case 11 and the second case 12. The configuration of the opposed walls is not limited to the configuration in which the opposed walls are opposed to each other in the inward/outward direction, and a configuration may be employed in which the opposed walls are opposed to each other in the circumferential direction. Alternatively, the first connection portion 60A and the second connection portion 70A may be provided with respective opposed walls having mutually matching shapes. Alternatively, a configuration may be employed in which the first case 11 and the second case are mutually positioned via the support frame 50A. In this case, the contact surface between the first case 11 and the support frame 50A serves as an opposed wall, and the contact surface between the second case 12 and the support frame 50A also serves as an opposed wall. Furthermore, the configuration of the opposed walls is not particularly limited, and the opposed walls may be implemented by, for example, mutual-contact surfaces between a cut and a protrusion having mutually matching shapes.

The second insertion groove 95 has a groove width (dimension in the horizontal direction) larger than the groove width of the first insertion groove 91. More specifically, the second insertion groove 95 is designed such that the groove width thereof is approximately equal to a dimension obtained by summing the groove width dimension of the first insertion groove 91 and the thickness dimension in the horizontal direction of the outer peripheral wall 92 on the lower side. Therefore, when the first case 11 and the second case 12 are assembled such that the inner peripheral wall 90 and the inner peripheral wall 94 are directly opposed to each other, the outer peripheral wall 92 on the lower side is fitted to an outer part in the second insertion groove 95 on the upper side, and the first insertion groove 91 and the second insertion groove 95 form an accommodation space 98 having a rectangular shape. In the pressure measuring chamber 1A, the support frame 50A is accommodated and held inside the accommodation space 98. The accommodation space 98 has a slightly larger dimension in the upward/downward direction than the support frame 50A.

An assembly procedure for the pressure measuring chamber 1A having the above configuration is as follows. First, the diaphragm 15 is mounted from above onto the first case 11 with the opening 21 thereof being oriented upward. That is, the lower surfaces of the flange portion 42A and the support frame 50A of the diaphragm 15 are located to be opposed to the first circling portion 23 of the first case 11. At this time, a lower portion of the support frame 50A of the diaphragm 15 is fitted into the first insertion groove 91 of the first case 11. In addition, the externally-extending portion 80 of the flexible membrane 40A of the diaphragm 15 is placed on the upper end surface 90a of the inner peripheral wall 90 of the first case 11.

Next, the second case 12 with the opening 31 thereof being oriented downward is mounted from above onto the first case 11. At this time, the outer peripheral wall 92 of the first case 11 and the support frame 50A are fitted into the second insertion groove 95 of the second case 12. As a result of performing the mounting in this manner, the support frame 50A is accommodated and held inside the accommodation space 98 formed by the first insertion groove 91 and the second insertion groove 95. At this time, the externally-extending portion (seal portion) 80 of the flange portion 42A of the diaphragm 15 is sandwiched and compressed between the upper end surface 90a of the inner peripheral wall 90 of the first case 11 and the lower end surface 94a of the inner peripheral wall 94 of the second case 12. In addition, the rib 94b protruding from the lower end surface 94a of the inner peripheral wall 94 bites into the upper surface of the externally-extending portion 80. That is, since the first case 11 and the second case 12 are combined in a state where the diaphragm 15 is sandwiched therebetween, the externally-extending portion 80 of the diaphragm 15 is sandwiched between the upper end surface 90a and the lower end surface 94a so that the support frame 50A is fixed between the first connection portion 60A and the second connection portion 70A (in FIG. 8B, inside the accommodation space 98). The support frame 50A may be fixed with a clearance from the first case 11 and the second case 12. Alternatively, the support frame 50A may be sandwiched and sealed between the first case 11 and the second case 12.

In this state, the first case 11 and the second case 12 are connected through adhesion or welding. For example, the upper end of the outer peripheral wall 92 of the first case 11 and the bottom surface (ceiling surface) of the second insertion groove 95 of the second case 12 opposed to this upper end in the upward/downward direction are connected through ultrasonic welding or the like. At this time, the protrusion 92a formed on the upper end surface of the outer peripheral wall 92 is melted so that the first case 11 and the second case 12 are welded together. As a result, the first case 11 is engaged with and fixed to the second case 12.

Such a pressure measuring chamber 1A according to embodiment 2 exhibits the same advantageous effects as those of the pressure measuring chamber 1 according to embodiment 1. That is, since the diaphragm 15 is composed of the flexible membrane 40A having a high flexibility and the support frame 50A formed from a material having a higher rigidity than the flexible membrane 40A, the stability of the shape of the diaphragm 15 is high. Therefore, the pressure measuring chamber 1A is excellent in terms of ease of assembly and can be appropriately assembled.

### (Modification 1)

Modification 1 of the structure regarding ease of assembly will be described. This modification 1 is applicable to, for example, the pressure measuring chamber 1 according to embodiment 1 or the pressure measuring chamber 1A according to embodiment 2. Here, description will be given regarding a case where this modification is applied to the pressure measuring chamber 1A according to embodiment 2.

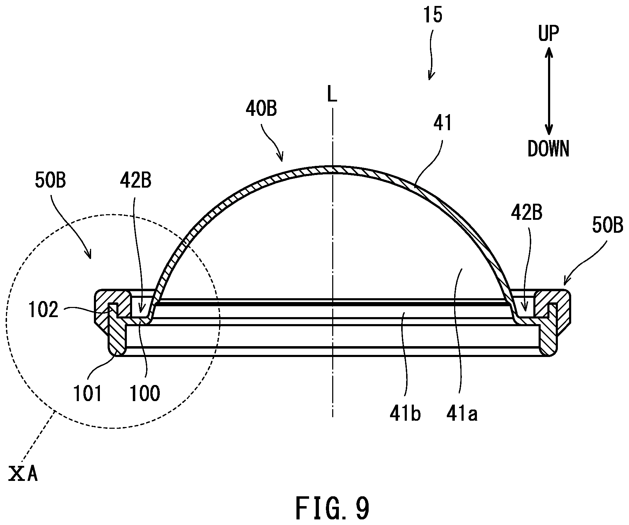

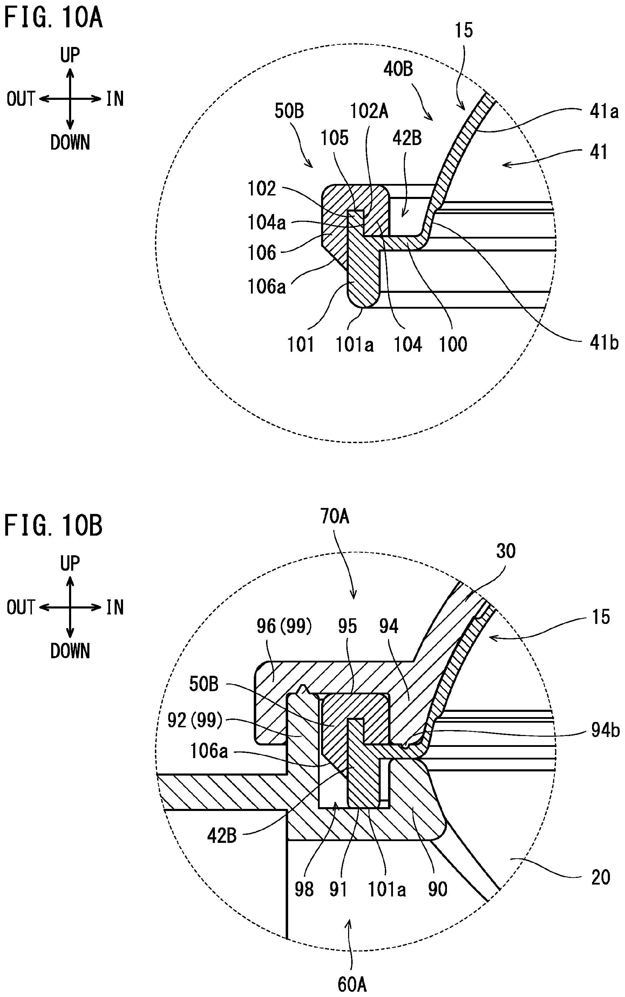

FIG. 9 is a cross-sectional view of a diaphragm 15 according to modification 1. FIG. 10A is an enlarged cross-sectional view of a part of the diaphragm 15 according to modification 1, and FIG. 10B is an enlarged cross-sectional view of a part of a pressure measuring chamber mounted with the diaphragm 15 according to modification 1. In the structure according to modification 1, constituents other than the diaphragm 15 are identical to those in embodiment 2. Therefore, the difference between the diaphragm 15 according to embodiment 2 and the diaphragm 15 according to modification 1 will be mainly described here.

As shown in FIG. 9, the diaphragm 15 includes a flexible membrane 40B and a support frame 50B, has a dome shape on the whole, and has a structure rotationally symmetric about the predetermined imaginary line L extending in the upward/downward direction. The flexible membrane 40B of the diaphragm 15 has: a membrane portion 41 composed of the dome-shaped body portion 41a and the small-thickness portion 41b; and a flange portion 42B. Out of these constituents, the membrane portion 41 is the same constituent as the membrane portion 41 according to each of embodiments 1 and 2. The above imaginary line L is a line passing through the center of the dome-shaped membrane portion 41.

The flange portion 42B has an externally-extending portion (seal portion) 100 extending outward from the other end of the small-thickness portion 41b. The externally-extending portion 100 has a substantially horizontal plate shape and circles along the small-thickness portion 41b to have an annular shape. The externally-extending portion 100 has an outer part having a lower surface from which a protrusion portion 101 extends downward. That is, the protrusion portion 101 is provided on a side, of the flange portion 42B, that is close to the first case 11. In addition, the externally-extending portion 100 has an outer end having an upper surface from which a to-be-engaged portion 102 extends upward.

The protrusion portion 101 extending downward has a vertically-elongated rectangular cross-sectional shape with the dimension thereof in the upward/downward direction being larger than the dimension thereof in the horizontal direction and has a lower end 101a having such an arc shape as to be bulged downward. The to-be-engaged portion 102 extending upward has a vertically-elongated rectangular shape with the dimension thereof in the upward/downward direction being larger than the dimension thereof in the horizontal direction. The dimension in the horizontal direction and the dimension in the upward/downward direction of the to-be-engaged portion 102 are smaller than those of the protrusion portion 101. The outer peripheral surface of the to-be-engaged portion 102 is flush with the outer peripheral surface of the protrusion portion 101, and the inner peripheral surface of the to-be-engaged portion 102 serves as a to-be-engaged surface 102a oriented inward.

Meanwhile, the support frame 50B has an annular shape on the whole, has a cross section having a U shape inverted upside down (i.e., inverted U shape), and is provided on a side, of the flange portion 42B, that is close to the second case 12 (i.e., upper side) opposite to the side on which the protrusion portion 101 is provided. Specifically, the support frame 50B has an inner frame 104, an outer frame 106, and an engagement groove 105 located therebetween. The inner frame 104 has a rectangular cross-sectional shape and is in contact with the upper surface of the externally-extending portion 100 and the inner peripheral surface (i.e., to-be-engaged surface 102a) of the to-be-engaged portion 102. The surface, of the inner frame 104, in contact with the to-be-engaged surface 102a serves as an engaging surface 104a oriented outward.

The outer frame 106 has a rectangular cross-sectional shape and has a lower surface and an outer surface which are connected at a portion at which a tapered surface 106a is formed. The tapered surface 106a is a surface tilted such that a portion thereof further away from the inner side toward the outer side is located on a further upper side. The outer frame 106 is in contact with outer peripheral surfaces of the flange portion 42B, i.e., the outer peripheral surface of the protrusion portion 101 and the outer peripheral surface of the to-be-engaged portion 102. The engagement groove 105 is formed between the inner frame 104 and the outer frame 106 and has a space having a rectangular cross-sectional shape substantially matching the cross-sectional shape of the to-be-engaged portion 102.

In the same manner as in the description of embodiment 2, such a diaphragm 15 according to modification 1 is sandwiched and fixed between the first connection portion 60A of the first case 11 and the second connection portion 70A of the second case 12. At this time, the externally-extending portion (seal portion) 100 of the diaphragm 15 is sandwiched and compressed between the inner peripheral wall 90 of the first case 11 and the inner peripheral wall 94 of the second case 12, and the rib 94b bites into the upper surface of the externally-extending portion 100. Consequently, the housing 14 is sealed between the inside and the outside thereof.

In addition, when the diaphragm 15 according to modification 1 is sandwiched between the first case 11 and the second case 12, the lower end 101a of the protrusion portion 101 is in pressure contact with the bottom surface of the first insertion groove 91. The protrusion portion 101 is a part of the flexible membrane 40B and is formed from a material having a lower rigidity and a higher flexibility than the support frame 50B. Therefore, the protrusion portion 101 having been brought into pressure contact is deformed and is in close contact with the bottom surface of the first insertion groove 91 over a wide range, whereby the sealability between the inside and the outside of the housing 14 is increased. Since the protrusion portion 101 is in contact with the first insertion groove 91 (i.e., first case 11), this contact particularly contributes to improvement of the airtightness and the liquid-tightness of the first chamber 13a. Therefore, blood flowing through the first chamber 13a can be effectively prevented from leaking.

Meanwhile, the diaphragm 15 according to modification 1 includes the highly rigid support frame 50B, and thus, similar to embodiments 1 and 2, the stability of the shape of the diaphragm 15 is high, and ease of assembly is excellent. In addition, the tapered surface 106a is provided on the outer side of the lower end of the support frame 50B as described above. Therefore, when the diaphragm 15 is mounted to the first case 11, the support frame 50B is easily fitted into the first insertion groove 91. In this respect as well, the structure according to modification 1 achieves improvement of ease of assembly.

### (Modification 2)

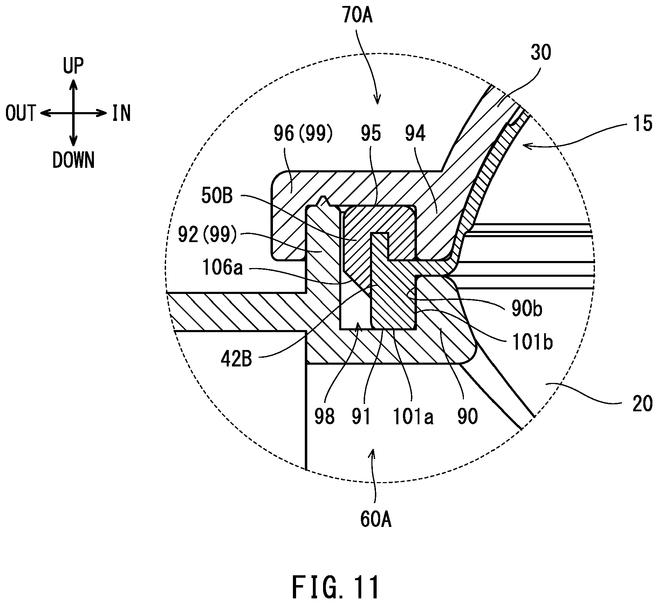

Although a configuration in which two portions, i.e., the rib 94b and the protrusion portion 101, allow the pressure measuring chamber to be sealed between the inside and the outside thereof has been employed in modification 1, no limitation thereto is made. FIG. 11 is an enlarged cross-sectional view of a part of a pressure measuring chamber according to modification 2. As shown in FIG. 11, the pressure measuring chamber according to modification 2 does not include the rib 94b, unlike in the configuration according to modification 1. In this configuration as well, the lower end 101a of the protrusion portion 101 is in pressure contact with the bottom surface of the first insertion groove 91, whereby the sealability between the inside and the outside of the pressure measuring chamber is ensured.

Furthermore, in the pressure measuring chamber according to modification 2, the dimension in the inward/outward direction of the protrusion portion 101 is larger than in the configuration according to modification 1. Consequently, when the diaphragm 15 is sandwiched between the first case 11 and the second case 12, the protrusion portion 101 is compressed in the upward/downward direction to have an increased dimension in the inward/outward direction. As a result, the lower end 101a of the protrusion portion 101 is in pressure contact with the bottom surface of the first insertion groove 91, and an inner end surface 101b of the protrusion portion 101 is also in pressure contact with an outer end surface 90b of the inner peripheral wall 90 of the first case 11. Therefore, even when the rib 94b is not provided, sealing can be assuredly performed at the two portions.

### (Modification 3)

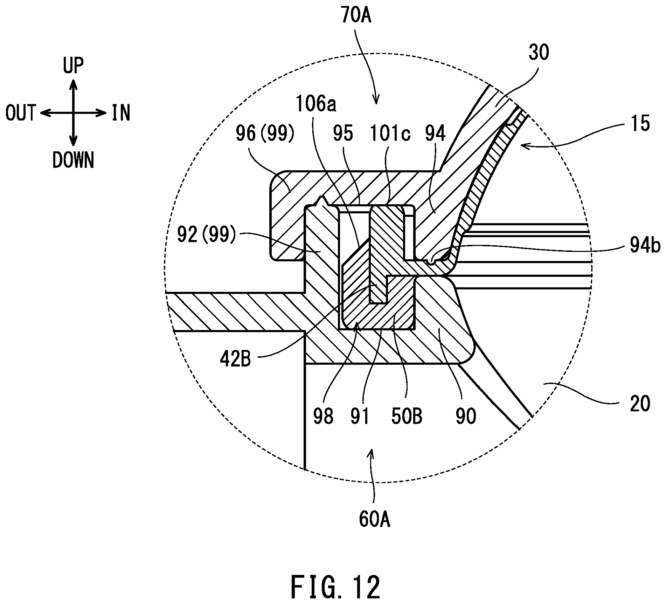

Although a configuration in which the protrusion portion 101 and the support frame 50B are respectively provided on the lower side close to the first case 11 and the upper side close to the second case 12 has been employed in modification 1, no limitation thereto is made. FIG. 12 is an enlarged cross-sectional view of a part of a pressure measuring chamber according to modification 3. Unlike the pressure measuring chamber according to modification 1, the pressure measuring chamber according to modification 3 has a configuration in which the protrusion portion 101 and the support frame 50B are respectively provided on the upper side close to the second case 12 and the lower side close to the first case 11. The other constituents are the same as those in modifications 1 and 2.

In the case of such modification 3, the protrusion portion 101 has an upper end 101c in close contact with the bottom surface (ceiling surface) of the second insertion groove 95 over a wide range. Therefore, the sealability between the inside and the outside of the housing 14 is ensured by two portions, i.e., the rib 94b and the upper end 101c of the protrusion portion 101. Furthermore, in the case of modification 3, ease of assembly of the pressure measuring chamber becomes favorable. That is, when the diaphragm 15 is disposed (mounted) on the first case 11, the highly rigid support frame 50B of the diaphragm 15 is disposed in the first insertion groove 91 of the first case 11. Therefore, the diaphragm 15 can be easily and stably positioned with respect to the first case 11.

In the configuration shown in FIG. 12 as well, the rib 94b may be dispensed with as described in modification 2. Also, along with the dispensing with the rib 94b, the dimension in the inward/outward direction of the protrusion portion 101 may be increased so that the protrusion portion 101 is, at two portions (i.e., the upper end and the inner end surface) thereof, brought into contact with the second insertion groove 95.

### (Modification 4)

Modification 4 of the structure regarding ease of assembly will be described. This modification 4 is also applicable to, for example, the pressure measuring chamber 1 according to embodiment 1 or the pressure measuring chamber 1A according to embodiment 2. Here, description will be given regarding a case where this modification is applied to the pressure measuring chamber 1A according to embodiment 2.

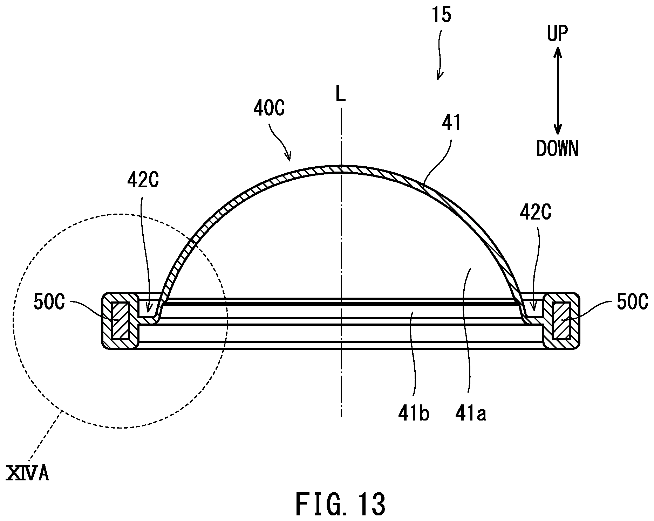

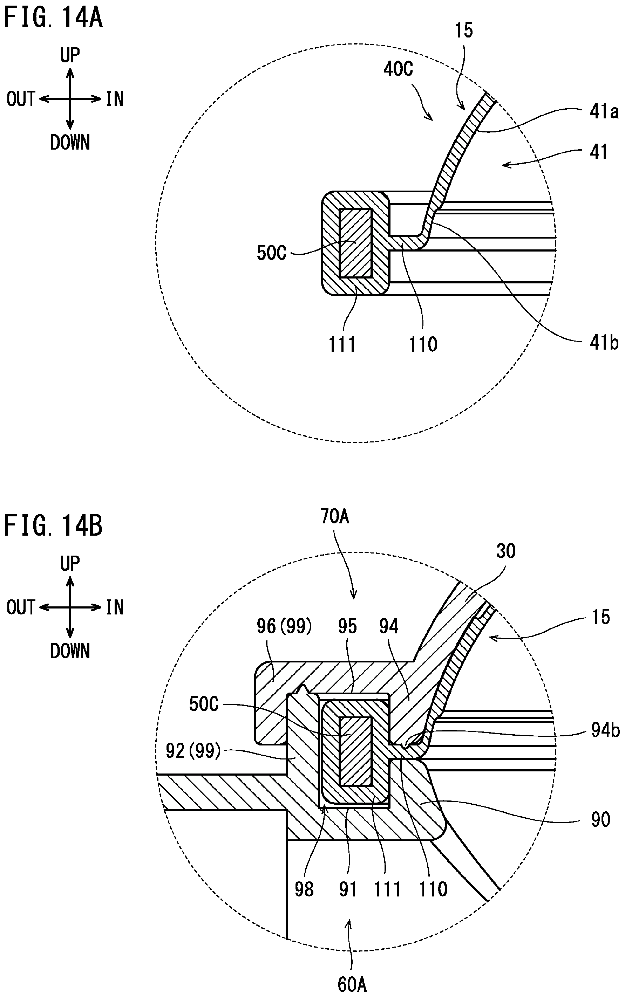

FIG. 13 is a cross-sectional view of a diaphragm 15 according to modification 4. FIG. 14A is an enlarged cross-sectional view of a part of the diaphragm 15 according to modification 4, and FIG. 14B is an enlarged cross-sectional view of a part of a pressure measuring chamber mounted with the diaphragm 15 according to modification 4. In the structure according to modification 4, constituents other than the diaphragm 15 are identical to those in embodiment 2. Therefore, the difference between the diaphragm 15 according to embodiment 2 and the diaphragm 15 according to modification 4 will be mainly described here.

As shown in FIG. 13, the diaphragm 15 includes a flexible membrane 40C and a support frame 50C, has a dome shape on the whole, and has a structure rotationally symmetric about the predetermined imaginary line L extending in the upward/downward direction. The flexible membrane 40C of the diaphragm 15 has: a membrane portion 41 composed of the dome-shaped body portion 41a and the small-thickness portion 41b; and a flange portion 42C. Out of these constituents, the membrane portion 41 is the same constituent as the membrane portion 41 according to each of embodiments 1 and 2 and modification 1. The above imaginary line L is a line passing through the center of the dome-shaped membrane portion 41.

The flange portion 42C has an externally-extending portion (seal portion) 110 extending outward from the other end of the small-thickness portion 41b. The externally-extending portion 110 has a substantially horizontal plate shape and circles along the small-thickness portion 41b to have an annular shape. The externally-extending portion 110 has an outer end provided with an enlarged portion 111. The enlarged portion 111 has a vertically-elongated rectangular shape with the dimension thereof in the upward/downward direction being larger than the dimension thereof in the horizontal direction, and has corners each chamfered into an arc shape. The cross-sectional dimensions of the enlarged portion 111 are approximately equal to the cross-sectional dimensions of the rectangular accommodation space 98 formed when the first case 11 and the second case 12 are mounted. Strictly speaking, the enlarged portion 111 has a size obtained by slightly reducing the accommodation space 98 in both the vertical and horizontal directions.

Meanwhile, the support frame 50C has an annular shape on the whole and has a vertically-elongated rectangular cross-sectional shape with the dimension thereof in the upward/downward direction being larger than the dimension thereof in the horizontal direction. In addition, the cross-sectional dimensions in both the upward/downward direction and the horizontal direction of the support frame 50C are smaller than those of the enlarged portion 111 of the flange portion 42C. Such a support frame 50C is embedded in the enlarged portion 111. That is, as shown in the cross-sectional view in FIG. 14B, the support frame 50C is enclosed by the enlarged portion 111 in all of the inward, outward, upward, and downward directions.

In the same manner as in the description of embodiment 2, such a diaphragm 15 according to modification 4 is sandwiched and fixed between the first connection portion 60A of the first case 11 and the second connection portion 70A of the second case 12. At this time, the externally-extending portion (seal portion) 110 of the diaphragm 15 is sandwiched and compressed between the inner peripheral wall 90 of the first case 11 and the inner peripheral wall 94 of the second case 12, and the rib 94b bites into the upper surface of the externally-extending portion 110. Consequently, the housing 14 is sealed between the inside and the outside thereof. In addition, in the state where the externally-extending portion 110 of the diaphragm 15 is sandwiched in this manner, the support frame 50C is accommodated and fixed between the first connection portion 60A and the second connection portion 70A (in FIG. 14B, inside the accommodation space 98). The enlarged portion 111 may be fixed with a clearance from the first case 11 and the second case 12. Alternatively, the enlarged portion 111 may be sandwiched and sealed between the first case 11 and the second case 12. In this case, the rib 94b may be dispensed with.

Meanwhile, the diaphragm 15 according to modification 4 includes the highly rigid support frame 50C, and thus, similar to embodiments 1 and 2 and modifications 1 to 3, the stability of the shape of the diaphragm 15 is high, and ease of assembly is excellent. In addition, the corners of the enlarged portion 111 of the flange portion 42C are rounded as described above. Consequently, when the diaphragm 15 is mounted on the first case 11, the enlarged portion 111 of the flange portion 42C is easily fitted into the first insertion groove 91. Furthermore, when the second case 12 is placed as well, the enlarged portion 50C is easily fitted into the second insertion groove 95. In this respect as well, the structure according to modification 4 achieves improvement of ease of assembly.

### (Embodiment 3)

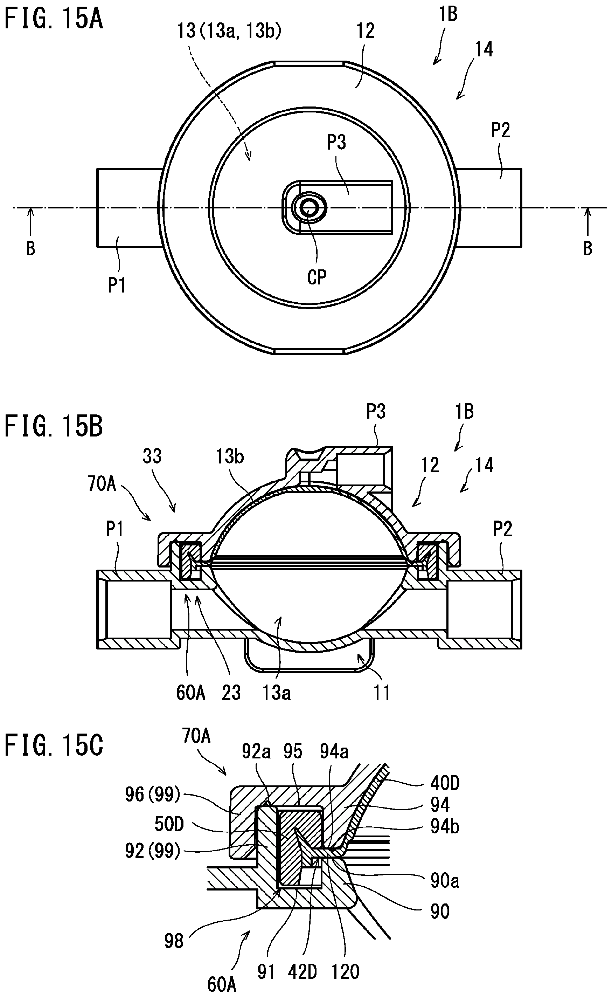

A configuration of a pressure measuring chamber 1B according to embodiment 3 of the present disclosure will be described. FIG. 15A is a plan view showing the appearance of the configuration of the pressure measuring chamber 1B, FIG. 15B is a cross-sectional view taken along the line B-B in FIG. 15A, and FIG. 15C is an enlarged view which is a part of the cross-sectional view in FIG. 15B. Hereinafter, constituents of the pressure measuring chamber 1B that differ from those of the pressure measuring chamber 1A according to embodiment 2 will be mainly described.

The pressure measuring chamber 1B has the housing 14 in which the chamber space 13 is formed by combining the first case 11 and the second case 12. Inside the housing 14, a flexible diaphragm 15 is provided and partitions the chamber space 13 into the first chamber 13a on the first case 11 side and the second chamber 13b on the second case 12 side. The cross-sectional view in FIG. 15B shows a configuration in which the diaphragm 15 is accommodated in a state of being bulged upward in a protruding shape.

The same first port P1 and second port P2 as those in embodiment 2 are formed in the first case 11, and the same third port P3 as that in embodiment 2 is formed in the second case 12 as well. The flow paths of these two first and second ports P1 and P2 are coaxial with each other and have center lines passing through a center CP of the chamber space 13 and extending in the frontward/rearward direction.

Alternatively, the first port P1 and the second port P2 may be provided such that the axial positions thereof are offset from each other leftward/rightward or such that the axial positions thereof intersect with each other or are parallel to each other.

Meanwhile, in the pressure measuring chamber 1B according to the present disclosure as well, the diaphragm 15 is composed of a flexible membrane 40D formed from a highly flexible material and a support frame 50D formed from a material having a higher rigidity than the flexible membrane 40D, whereby improvement of ease of assembly and the like are achieved. Hereinafter, such a structure regarding ease of assembly will be described. In the housing 14, the first circling portion 23 of the first case 11 and the second circling portion 33 of the second case 12 are respectively provided with the first connection portion 60A and the second connection portion 70A in order to support the diaphragm 15.

The configuration of the first connection portion 60A and the second connection portion 70A is similar to the configuration described in embodiment 2. That is, as shown in FIG. 15C, the first connection portion 60A has the inner peripheral wall 90, the outer peripheral wall 92, the first insertion groove 91, the upper end surface 90a, the protrusion 92a, etc., and the second connection portion 70A has the inner peripheral wall 94, the outer peripheral wall 96, the second insertion groove 95, the lower end surface 94a, the rib 94b, etc. These constituents have been described in detail in embodiment 2 and thus will not be described here (see FIG. 8B, FIG. 10B, FIG. 11, FIG. 12, and FIG. 14B as well).

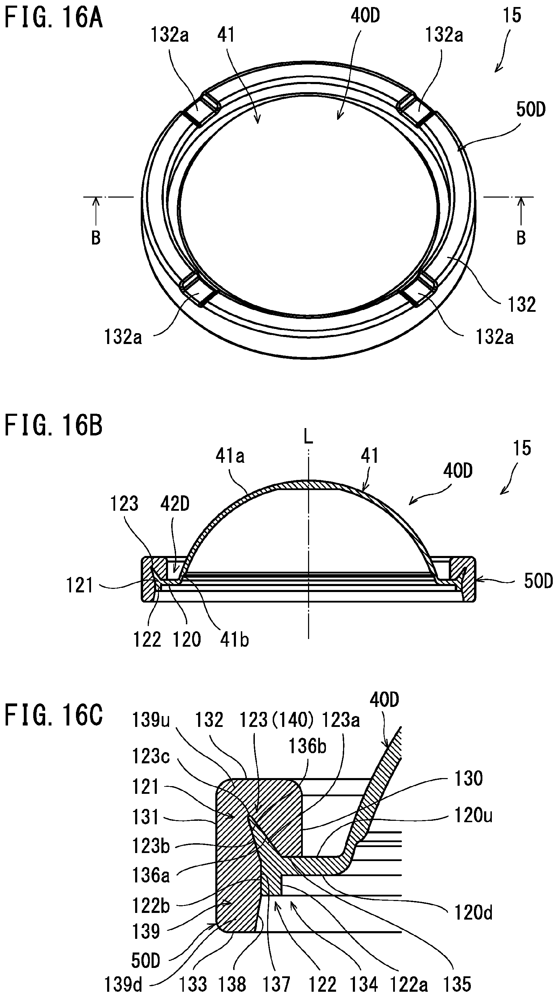

FIG. 16A is a perspective view of the diaphragm 15 according to embodiment 3, FIG. 16B is a cross-sectional view, taken along the line B-B, of the diaphragm 15 in FIG. 16A, and FIG. 16C is an enlarged view which is a part of the cross-sectional view in FIG. 16B. The diaphragm 15 includes the flexible membrane 40D and the support frame 50D, and the flexible membrane 40D has: a membrane portion 41 composed of the dome-shaped body portion 41a and the small-thickness portion 41b; and a flange portion 42D. Out of these constituents, the membrane portion 41 is the same constituent as the membrane portion 41 according to each of embodiments 1 and 2.

The flange portion 42D has an externally-extending portion (seal portion) 120 extending outward from the other end of the small-thickness portion 41b. The externally-extending portion 120 has a substantially horizontal plate shape and circles along the small-thickness portion 41b to have an annular shape. The externally-extending portion 120 has an outer end provided with an enlarged portion 121. The enlarged portion 121 has: a lower enlarged portion 122 extending downward from a lower surface 120d of the outer end of the externally-extending portion 120 and circling along this outer end; and an upper enlarged portion (embedded portion) 123 extending upward from an upper surface 120u of the outer end of the externally-extending portion 120 and circling along this outer end in the same manner.

The lower enlarged portion 122 has a thickness dimension which is the dimension between an inner surface 122a close to the imaginary line L and an outer surface 122b far from the imaginary line L, and the thickness dimension is substantially unchanged from the upper end to the lower end. The lower enlarged portion 122 has a rectangular cross-sectional shape with the dimension thereof in the upward/downward direction and the thickness dimension thereof being approximately equal to each other. In addition, both the inner surface 122a and the outer surface 122b are surfaces parallel to the imaginary line L and substantially perpendicular to the lower surface 120d of the externally-extending portion 120.

The upper enlarged portion 123 has a wedge-like cross-sectional shape. Specifically, the upper enlarged portion 123 has: an inner tilted surface 123a extending upward and outward from the upper surface 120u of the externally-extending portion 120; and an outer tilted surface 123b extending upward and outward from the outer end surface of the externally-extending portion 120. The inner tilted surface 123a is tilted from the upper surface 120u of the externally-extending portion 120 at a larger angle than the outer tilted surface 123b. Therefore, the inner tilted surface 123a and the outer tilted surface 123b have respective upper ends connected to each other to form an upper end 123c of the upper enlarged portion 123. The upper end 123c is located further outward than (i.e., overhangs) the outer end of the externally-extending portion 120.

The upper enlarged portion 123 serves as an embedded portion embedded in the support frame 50D. That is, the upper enlarged portion 123 located at the outer end portion of the flange portion 42D of the flexible membrane 40D is embedded in the support frame 50D such that the inner tilted surface 123a as an inner surface in the inward/outward direction (second direction) and the outer tilted surface 123b as an outer surface in the inward/outward direction (second direction) are respectively fixed to an inner tilted surface 136a and an outer tilted surface 136b of the support frame 50D (see FIG. 16C). Consequently, large areas can be ensured for the fixation surfaces of the flexible membrane 40D and the support frame 50D, and the flexible membrane 40D is stably held by the support frame 50D.

The inner tilted surface 136a of the support frame 50D serves as an engaging surface, the inner tilted surface 123a of the flexible membrane 40D serves as a to-be-engaged surface, the inner tilted surface 123a is located on the outer side relative to the inner tilted surface 136a, and the inner tilted surfaces 136a and 123a are located to be opposed to each other in the inward/outward direction. In this manner, the inner tilted surface 136a of the support frame 50D corresponds to the engaging surface 53 in embodiment 1, the inner tilted surface 123a of the flexible membrane 40D corresponds to the to-be-engaged surface 47 in embodiment 1, and the same advantageous effects as those of these constituents in embodiment 1 are exhibited.

In addition, the enlarged portion 121 including this upper enlarged portion (embedded portion) 123 has an upper end and a lower end which are covered by the support frame 50D. Specifically, the support frame 50D has a protective portion 139 having, in the upward/downward direction (first direction), an upper extended portion 139u extending upward beyond the outer surface (upper surface) of the enlarged portion 121 and a lower extended portion 139d extending downward beyond the outer surface (lower surface) of the enlarged portion 121. Consequently, the outer peripheral edge of the flexible membrane 40D can be inhibited from coming into contact with wall surfaces of the first insertion groove 91 and the second insertion groove 95 during assembly.

Meanwhile, the support frame 50D has an annular shape on the whole, has a substantially rectangular cross-sectional shape opened downward and inward, and is provided to cover the enlarged portion 121 of the flange portion 42D. More specifically, the support frame 50D has an inner surface 130 and an outer surface 131 extending in the upward/downward direction, an upper surface 132 connecting the upper end of the inner surface 130 and the upper end of the outer surface 131, and a lower surface 133 extending inward from the lower end of the outer surface 131, and an opening portion 134 is formed between the inner surface 130 and the lower surface 133. A connection portion between the inner surface 130 and the upper surface 132, a connection portion between the outer surface 131 and the upper surface 132, and a connection portion between the outer surface 131 and the lower surface 133 are each rounded.

The opening portion 134 of the support frame 50D has a lower surface 135 extending outward from the lower end of the inner surface 130 along the upper surface 120u of the externally-extending portion 120. The opening portion 134 further has: the inner tilted surface 136a extending upward and outward from the outer end of the lower surface 135; and the outer tilted surface 136b extending downward and inward from the upper end of the inner tilted surface 136a. Out of these surfaces, the inner tilted surface 136a extends along the inner tilted surface 123a of the above upper enlarged portion 123, and the outer tilted surface 136b extends along the outer tilted surface 123b of the above upper enlarged portion 123. The opening portion 134 further has: a first inner surface 137 extending downward from the lower end of the outer tilted surface 136b along the outer surface 122b of the lower enlarged portion 122 of the externally-extending portion 120; and a second inner surface 138 extending to connect the lower end of the first inner surface 137 and the inner end of the lower surface 133. Out of these surfaces, the first inner surface 137 extends in the upward/downward direction along the outer surface 122b of the lower enlarged portion 122, and the second inner surface 138 is a tilted surface extending from the lower end of the first inner surface 137 so as to be tilted downward and outward.

The portion of the support frame 50D that is located on the outer side relative to the enlarged portion 121 of the flange portion 42 of the flexible membrane 40D (i.e., the portion between the outer surface 131 and each of the first inner surface 137, the second inner surface 138, and the outer tilted surface 136b) constitutes the protective portion 139. The protective portion 139 makes it possible to inhibit the flexible membrane 40D from rubbing on the wall surface of the first insertion groove 91 or the second insertion groove 95 when the diaphragm 15 is inserted into the accommodation space 98. In each of the diaphragms according to the already described embodiments and modifications as well, the portion of the support frame that is located on the outer side relative to the enlarged portion constitutes a protective portion and exhibits the same advantageous effects described above.

The flexible membrane 40D is connected to the support frame 50D by fitting the enlarged portion 121 into the opening portion 134 opened downward and inward in the support frame 50D. Meanwhile, the upper enlarged portion 123 of the flexible membrane 40D serves as a coming-off preventer 140 for preventing coming-off from the opening portion 134 of the support frame 50D. That is, the outer tilted surface 123b of the upper enlarged portion 123 of the flexible membrane 40D and the outer tilted surface 136b of the opening portion 134 of the support frame 50D are engaged (in contact) with each other in the upward/downward direction, whereby the flexible membrane 40D and the support frame 50D are prevented from being separated from each other.

As shown in FIG. 15C, such a diaphragm 15 is supported by sandwiching and compressing, between the upper end surface 90a of the first connection portion 60A of the first case 11 and the rib 94b on the lower end surface 94a of the second connection portion 70A of the second case 12, the externally-extending portion (seal portion) 120 of the flange portion 42D on the inner side relative to the support frame 50D. Meanwhile, the dimension in the upward/downward direction of the accommodation space 98 is longer than the dimension in the upward/downward direction of the support frame 50D. Therefore, the support frame 50D is accommodated inside the accommodation space 98 formed by the first insertion groove 91 of the first connection portion 60A and the second insertion groove 95 of the second connection portion 70A and is loosely fitted with a gap in the upward/downward direction. Consequently, the diaphragm 15 is sandwiched (supported) between the first case 11 and the second case 12 in an airtight and liquid-tight manner.

The dimension in the inward/outward direction of the accommodation space 98 is longer than the dimension in the inward/outward direction of the support frame 50D, and the support frame 50D is loosely fitted into the accommodation space 98 with a gap also in the inward/outward direction. Consequently, the support frame 50D is easily inserted into the accommodation space 98. Here, although the accommodation space 98 in FIG. 15C is presented as an example in which there are gaps on both the upper side and the lower side relative to the support frame 50D, no limitation to such a configuration is made. For example, a configuration may be employed in which the support frame 50D comes into contact with the internal bottom surface of the second insertion groove 91 owing to the own weight of the support frame 50D so that a gap is present only on the upper side relative to the support frame 50D. In addition, a configuration in which the dimension of the gaps in the upward/downward direction relative to the support frame 50D (the total value of the gap on the upper side and the gap on the lower side) is larger than the dimension of the gaps in the inward/outward direction relative to the support frame 50D (the total value of the gap on the inner side and the gap on the outer side) is preferable.

### (Molding Procedure for Diaphragm)

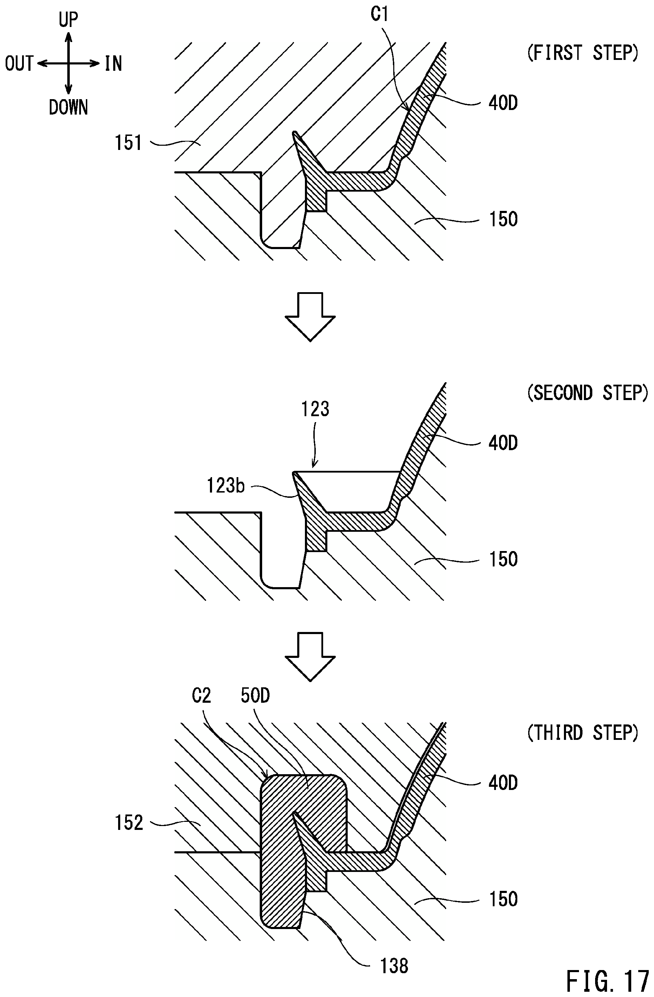

The above diaphragm 15 can be produced through, for example, two-shot injection molding. FIG. 17 is a schematic diagram showing an example of a molding process for the diaphragm 15. As shown in FIG. 17, in a first step, a lower mold 150 and a first upper mold 151 are combined from below and above, whereby a cavity C1 is formed. Then, a first resin is poured into the cavity C1 to mold the flexible membrane 40D. For example, a first gate is provided at a position corresponding to the uppermost portion of the dome-shaped membrane portion 41, and the first resin is poured from the first gate.

Next, in a second step, the lower mold 150 and the flexible membrane 40D are released from the first upper mold 151 and are rotated. Upon this releasing, the lower mold 150 and the first upper mold 151 are moved away in the upward/downward direction. In a subsequent third step, a second upper mold 152 is combined with the lower mold 150 in the upward/downward direction so as to sandwich the flexible membrane 40D therebetween, whereby a cavity C2 is formed. A second resin is poured into the cavity C2 to form the support frame 50D. For example, one or a plurality of second gates (in FIG. 16A, four second gates located at regular intervals in the circumferential direction) are provided at positions corresponding to the upper surface 132 of the support frame 50D, and the second resin is poured from the second gates. Lastly, the lower mold 150 and the second upper mold 152 are moved away in the upward/downward direction so that the diaphragm 15 is released.

Here, the outer tilted surface 123b of the upper enlarged portion 123 of the flexible membrane 40D has an undercut shape with respect to the first upper mold 151. However, the flexible membrane 40D has low hardness and is flexible, whereby the first upper mold 151 can be easily released in the second step. Meanwhile, since the outer tilted surface 123b has an undercut shape, the flexible membrane 40D and the support frame 50D are engaged with each other and are prevented from being separated from each other. As a material for forming the flexible membrane 40D, for example, an elastomer resin is usable. As a material for forming the support frame 50D, for example, polypropylene (PP) or polyvinyl chloride (PVC) is usable.

As shown in FIG. 16C, the second inner surface 138 of the support frame 50D is a tilted surface. This second inner surface 138 portion has a draft between the lower mold 150 and the first upper mold 151 (see the first step in FIG. 17). Therefore, the first upper mold 151 is smoothly released in the second step. In FIG. 16A, four recesses 132a regularly arranged in the circumferential direction are formed in the upper surface 132 of the support frame 50D.
The recesses 132a are sunk parts corresponding to the second gates from which the second resin is poured in the third step. That is, after the second upper mold 152 is released, burrs remain on portions, of the upper surface 132 of the support frame 50D, that correspond to the second gates. The recesses 132a as sunk parts are spaces for accommodating the burrs and prevent the burrs from coming into contact with outside components.

Meanwhile, although the upper enlarged portion 123 shown in FIG. 16C has a wedge-like cross-sectional shape, no limitation thereto is made. For example, the upper enlarged portion 123 may have a plate shape with a fixed thickness or may have still another shape. Also, although a shape extending upward and outward has been presented as an example of the shape of the upper enlarged portion 123, no limitation thereto is made. For example, a shape extending upward and inward may be employed. Even when the upper enlarged portion 123 has a shape extending upward and inward, the upper enlarged portion 123 functions as the coming-off preventer 140. Also, although the recesses 132a as sunk parts are provided in the upper surface 132 of the support frame 50D as shown in FIG. 16A, the recesses 132a may be dispensed with. Furthermore, the lower enlarged portion 122 of the enlarged portion 121 may be dispensed with from the viewpoint of preventing coming-off.

As can be understood from the above description, the opening portion 134 which belongs to the support frame 50D and which is to be engaged with the flexible membrane 40D is preferably opened downward in the case of molding the diaphragm 15 through injection molding. Consequently, the releasing direction at the time of molding can be set to be the upward/downward direction alone, whereby the molding can be simplified. In addition, the portion (the above coming-off preventer 140) that belongs to the flexible membrane 40D and that is to be engaged with the support frame 50D preferably has an undercut shape as seen in the upward/downward direction. Consequently, the undercut portion is engaged with the support frame 50D in the upward/downward direction identical to the opening direction of the opening portion 134 to function as a coming-off (separation) preventer.

Although a configuration in which the opening portion 134 of the support frame 50D is opened downward and the upper enlarged portion 123 of the flexible membrane 40D has an undercut shape has been presented as an example in the above description, this configuration may be turned upside down. That is, the pressure measuring chamber 1B may have a configuration in which the above diaphragm 15 is sandwiched between the first case 11 and the second case 12 in a state of being inverted upside down. Also, the movement direction in which the lower mold 150 and the first upper mold 151 or the second upper mold 152 are separated or combined has been described as being the upward/downward direction in relation to the molding procedure for the diaphragm 15. However, without limitation thereto, the molds may be moved in another direction such as the horizontal direction. Furthermore, separation or combination of the molds only has to involve relative movement between the lower mold 150 and the first upper mold 151 or the second upper mold, and, for example, the first upper mold 151 or the second upper mold 152 may be moved relative to the lower mold 150 in contrast to the above description.

### (Modification 5)

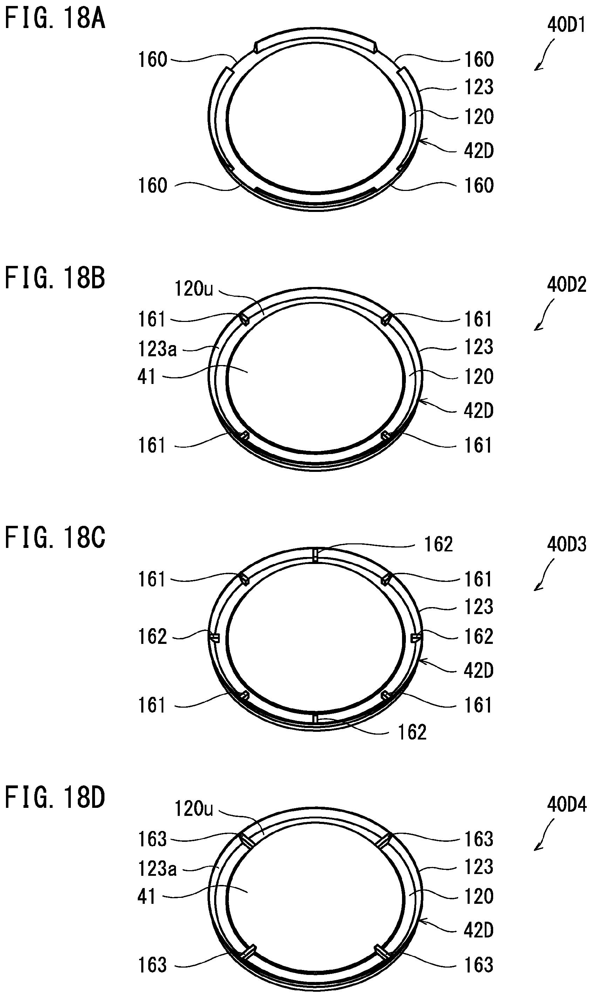

Meanwhile, regarding the pressure measuring chamber 1B according to embodiment 3, a procedure in which the resins are poured from above is employed for two-shot molding of the diaphragm 15 in order to simplify the process. In this case, when the second resin is poured from above in the third step, the poured second resin might come into contact with the upper enlarged portion 123 of the flexible membrane 40D molded in the first step, whereby the upper enlarged portion 123 might be deformed. When the upper enlarged portion 123 is deformed during pouring of the second resin, the second resin might flow in an unexpected manner, whereby a molding imperfection such as remaining of air might occur. In modification 5, four types of configurations (modifications) of a flexible membrane for preventing such a molding imperfection will be disclosed with reference to FIG. 18A to FIG. 18D. Hereinafter, only the constituents different from those of the above flexible membrane 40D will be described, and the other constituents are identical to those of the flexible membrane 40D.

In a flexible membrane 40D1 shown in FIG. 18A, cuts 160 are formed in parts of the upper enlarged portion 123. The cuts 160 are present at the positions corresponding to the second gates (i.e., the four positions corresponding to the recesses 132a of the support frame 50D), and the upper enlarged portion 123 is formed at none of the portions at which the cuts 160 are located in the externally-extending portion 120 of the flange portion 42D. Consequently, the second resin does not come into direct contact with the upper enlarged portion 123 immediately after being poured from the second gates in the third step, whereby deformation of the upper enlarged portion 123 can be prevented.

In a flexible membrane 40D2 shown in FIG. 18B, the upper enlarged portion 123 is formed over the entire periphery, and ribs 161 extending inward from the upper enlarged portion 123 are provided at the positions (here, four positions) corresponding to the second gates. The ribs 161 connect the inner tilted surface 123a of the upper enlarged portion 123 and the upper surface 120u of the externally-extending portion 120 and are not connected to the membrane portion 41. By having such ribs 161, the upper enlarged portion 123 comes to have an improved strength and can be prevented from being deformed even when the poured second resin comes into direct contact with the upper enlarged portion 123. In a case where the strength of the upper enlarged portion 123 can be sufficiently ensured by the ribs 161, the ribs 161 may be set at positions shifted from each other in the circumferential direction without being provided at the positions corresponding to the second gates.

A flexible membrane 40D3 shown in FIG. 18C has a configuration in which ribs 162 having the same configurations are further provided in addition to the ribs 161 in FIG. 18B. Thus, the number of the ribs may be equal to or different from the number of the second gates. By setting the number of the ribs to be larger than the number of the second gates as in the example in FIG. 18C, the strength of the upper enlarged portion 123 can be further improved, and deformation thereof can be more assuredly prevented.

A flexible membrane 40D4 shown in FIG. 18D has, for example, ribs 163 having configurations obtained by extending the ribs 161 in FIG. 18B further inward. That is, the ribs 163 connect the inner tilted surface 123a of the upper enlarged portion 123, the upper surface 120u of the externally-extending portion 120, and the upper surface (the surface on the second chamber 13b side) of the membrane portion 41. Consequently, the lengths of the connection sides of the ribs 163 are elongated, and the upper enlarged portion 123 can be more firmly supported. Therefore, the strength of the upper enlarged portion 123 can be improved, and deformation thereof can be prevented. Although FIG. 18D shows an example in which the number of the provided ribs 163 is equal to the number of the second gates, this is also merely an example, and less or more ribs 163 may be provided.

Each of the flexible membranes disclosed in the above respective embodiments and respective modifications is preferably a membrane that, after a positional shift, is not elastically restored to a state taken before the positional shift. That is, the flexible membrane is positionally shifted in the upward/downward direction according to the pressure difference between the first chamber (blood chamber) 13a and the second chamber (air chamber) 13b and, when the pressure difference is zero, retains the orientation taken upon the positional shift. For example, when the pressure in the second chamber 13b becomes lower than the pressure in the first chamber 13a, the membrane portion 41 of the flexible membrane comes to have a shape protruding to the second chamber 13b side. Then, even when the pressure difference between the first chamber 13a and the second chamber 13b becomes zero in this state, the membrane portion 41 retains the shape protruding to the second chamber 13b side. Likewise, when the pressure in the first chamber 13a becomes lower than the pressure in the second chamber 13b, the membrane portion 41 of the flexible membrane comes to have a shape protruding to the first chamber 13a side. Then, even when the pressure difference between the first chamber 13a and the second chamber 13b becomes zero in this state, the membrane portion 41 retains the shape protruding to the first chamber 13a side.

As such a flexible membrane, for example, a flexible membrane having the following properties is preferable. That is, out of the first port P1 and the second port P2 leading to the first chamber 13a, the first port P1 is closed, and the second port P2 is connected to a pressure sensor via a tube. The tube is branched at an intermediate portion thereof, and this branched portion is connected to an air syringe. The second chamber 13b is opened to atmospheric air. In this state, air is injected through the air syringe, and the pressure at which the membrane portion 41 starts to be positionally shifted owing to the injection is measured by the pressure sensor. The pressure which is measured through this method and which acts on the flexible membrane according to the present disclosure is preferably not more than 10 kPa and more preferably not more than 5 kPa. As such a flexible membrane, for example, a flexible membrane molded from an elastomer resin is suitably usable.

### Industrial Applicability

The present disclosure is suitably applicable to diaphragm-type pressure measuring chambers for measuring a pressure of blood flowing through a blood circuit.

### Reference Signs List

- 1: pressure measuring chamber
- 11: first case
- 12: second case
- 13: chamber space
- 13a: first chamber
- 13b: second chamber
- 14: housing
- 15: diaphragm
- 40: flexible membrane
- 41: membrane portion
- 42: flange portion
- 50: support frame
- 60: first connection portion
- 63: first insertion groove
- 70: second connection portion
- 73: second insertion groove
- 101: protrusion portion

## Claims

1. A diaphragm-type pressure measuring chamber for measuring a pressure of blood in a blood circuit, the diaphragm-type pressure measuring chamber comprising:
a first case having a peripheral portion provided with a first connection portion;
a second case forming a chamber space between the second case and the first case and having a peripheral portion provided with a second connection portion connected to the first connection portion; and
a flexible dome-shaped diaphragm partitioning the chamber space into a first chamber on a blood side and a second chamber on a pressure sensor side, wherein
the first chamber is formed between the first case and the diaphragm and is in communication with a blood flow path in the blood circuit,
the diaphragm has a flexible membrane deformable in a first direction according to a pressure difference between the first chamber and the second chamber and a support frame provided on a peripheral portion of the flexible membrane and fixed between the first connection portion and the second connection portion, and
the support frame has a higher rigidity than the flexible membrane.

2. The diaphragm-type pressure measuring chamber according to claim 1, wherein at least one of the first connection portion and the second connection portion is provided with an insertion groove into which the support frame is inserted in the first direction.

3. The diaphragm-type pressure measuring chamber according to claim 2, wherein
the first connection portion and the second connection portion form an accommodation space accommodating the support frame, and
the flexible membrane has a seal portion sandwiched and compressed between the first connection portion and the second connection portion.

4. The diaphragm-type pressure measuring chamber according to claim 3, wherein
the first case is engaged with the second case, and
the accommodation space is formed with a dimension thereof in the first direction being longer than a dimension in the first direction of the support frame to provide a gap between the support frame and the first connection portion or the second connection portion inside the accommodation space.

5. The diaphragm-type pressure measuring chamber according to claim 2, wherein the support frame has a protective portion located radially outward of the flexible membrane.

6. The diaphragm-type pressure measuring chamber according to claim 5, wherein the flexible membrane has an embedded portion having an inner surface and an outer surface in a second direction and embedded in the support frame with the inner surface and the outer surface being fixed to the support frame.

7. The diaphragm-type pressure measuring chamber according to claim 6, wherein the protective portion has an extended portion extending in the first direction beyond the outer surface of the embedded portion of the flexible membrane.

8. A diaphragm-type pressure measuring chamber for measuring a pressure of blood in a blood circuit, the diaphragm-type pressure measuring chamber comprising:
a first case having a peripheral portion provided with a first connection portion;
a second case forming a chamber space between the second case and the first case and having a peripheral portion provided with a second connection portion connected to the first connection portion; and
a flexible diaphragm partitioning the chamber space into a first chamber on a blood side and a second chamber on a pressure sensor side, wherein
the first chamber is formed between the first case and the diaphragm and is in communication with a blood flow path in the blood circuit,
the diaphragm has
a flexible membrane having a surface area larger than a cross-sectional area of the chamber space and having a three-dimensional shape at least partially protruding in a first direction, the flexible membrane being deformable in the first direction according to a pressure difference between the first chamber and the second chamber, and
a support frame provided on a peripheral portion of the flexible membrane and fixed between the first connection portion and the second connection portion, and
the support frame has a higher rigidity than the flexible membrane.
